# EUROPÄISCHE PATENTSCHRIFT

(11) **EP 3 556 847 B1**
(45) Veröffentlichungstag und Bekanntmachung des Hinweises auf die Patenterteilung: **03.03.2021**
(21) Anmeldenummer: 19175665.9
(22) Anmeldetag: 11.12.2015
(51) Int. Cl.: C12N 1/20, C12P 21/00, C12N 9/10, C12N 9/12

(54) **MIKROORGANISMENSTAMM UND VERFAHREN ZUR ANTIBIOTIKAFREIEN, FERMENTATIVEN HERSTELLUNG VON NIEDERMOLEKULAREN SUBSTANZEN UND PROTEINEN**
MICROORGANISM STRAIN AND METHOD FOR THE FERMENTATIVE PRODUCTION OF LOW MOLECULAR WEIGHT ANTIBIOTIC-FREE SUBSTANCES AND PROTEINS
SOUCHE DE MICRO-ORGANISMES ET PROCÉDÉ DE FABRICATION PAR FERMENTATION, SANS ANTIBIOTIQUES, DE SUBSTANCES ET DE PROTÉINES À FAIBLE POIDS MOLÉCULAIRE

(43) Veröffentlichungstag der Anmeldung: 23.10.2019
(62) Teilanmeldung aus: 15816123.2
(73) Patentinhaber: Wacker Chemie AG, 81737 München (DE)
(72) Erfinder: BORNHÖVD, Carsten, 81825 München (DE); THÖN, Marcel, 06114 Halle (DE)
(74) Vertreter: Fritz, Helmut

(56) Entgegenhaltungen:
- HAGG PETER ET AL: "A host/plasmid system that is not dependent on antibiotics and antibiotic resistance genes for stable plasmid maintenance in Escherichia coli", JOURNAL OF BIOTECHNOLOGY, ELSEVIER SCIENCE PUBLISHERS, AMSTERDAM, NL, Bd. 111, Nr. 1, 1. Juli 2004 (2004-07-01), Seiten 17-30, XP002316099, ISSN: 0168-1656, DOI: 10.1016/J.JBIOTEC.2004.03.010
- Régis Sodoyer ET AL: "Antibiotic-Free Selection for Bio-Production: Moving Towards a New "Gold Standard"" In: "Antibiotic-Free Selection for Bio-Production: Moving Towards a New "Gold Standard"", 4. April 2012 (2012-04-04), XP055270667, ISBN: 978-953-51-0472-8 DOI: 10.5772/28510, * Einleitung; Seite 537-539, Absatz 5.3. *
- BABA TOMOYA ET AL: "Construction of Escherichia coli K-12 in-frame, single-gene knockout mutants: the Keio collection", MOLECULAR SYSTEMS BIOLOGY, THE MACMILLAN BUILDING, LONDON, GB, Bd. 2, 1. Februar 2006 (2006-02-01), XP002519600, ISSN: 1744-4292, DOI: 10.1038/MSB4100050 [gefunden am 2006-02-21] -& Tomoya Baba ET AL: "Construction of Escherichia coli K-12 in-frame, single-gene knockout mutants: the Keio collection. Supplementary Table 6. E. coli K-12 essential gene candidates.", , 1. Januar 2006 (2006-01-01), XP055271173, Gefunden im Internet: URL:http://msb.embopress.org/content/msb/2 /1/2006.0008/DC8/embed/inline-supplementar y-material-8.xls?download=true [gefunden am 2016-05-09] -& Tomoya Baba ET AL: "Construction of Escherichia coli K-12 in-frame, single-gene knockout mutants: the Keio collection. Supplementary Table 7. COG classification of E. coli K-12 genes", , 1. Januar 2006 (
- DATSENKO KIRILL A ET AL: "One-step inactivation of chromosomal genes in Escherichia coli K-12 using PCR products", PROCEEDINGS OF THE NATIONAL ACADEMY OF SCIENCES, NATIONAL ACADEMY OF SCIENCES, US, Bd. 97, Nr. 12, 6. Juni 2000 (2000-06-06), Seiten 6640-6645, XP002210218, ISSN: 0027-8424, DOI: 10.1073/PNAS.120163297
- SAKAMOTO H ET AL: "Structure-function relationships of UMP kinases from pyrH mutants of Gram-negative bacteria", MICROBIOLOGY, SOCIETY FOR GENERAL MICROBIOLOGY, GB, Bd. 150, Nr. Part 7, 1. Juli 2004 (2004-07-01), Seiten 2153-2159, XP002349080, ISSN: 1350-0872, DOI: 10.1099/MIC.0.26996-0

## Beschreibung

Die Erfindung betrifft einen Mikroorganismenstamm und ein Verfahren zur antibiotikafreien fermentativen Herstellung von niedermolekularen Substanzen und Proteinen Die Erfindung ist in dem beigefügten Anspruchssatz aufgeführt.

Prinzipiell hat die natürliche Produktion von niedermolekularen Substanzen mit Hilfe von Mikroorganismen zugenommen und auch der Markt für rekombinante Proteinpharmazeutika ("Biologics") ist in den letzten Jahren stark gewachsen. Aufgrund des starken Kostendrucks bei der fermentativen Produktion, insbesondere für Pharmawirkstoffe auf Proteinbasis, wird laufend nach effizienteren und damit kostengünstigeren Verfahren und Systemen für deren Herstellung gesucht. Als Produzenten können verschiedene Mikroorganismen, wie Bakterien, Hefen, filamentöse Pilze oder auch Pflanzenzellen oder Tierzellen verwendet werden. Wirtschaftlich ausschlaggebend ist hierbei eine kostengünstige Fermentation, hohe Produktausbeuten und bei Proteinen eine korrekte Faltung bzw. Modifikation, die zu einem funktionellen Protein führt. Aufgrund seiner sehr gut untersuchten Genetik und Physiologie, der kurzen Generationszeit und der einfachen Handhabung, ist das Gram-negative Enterobakterium *Escherichia coli (E. coli)* gegenwärtig der am häufigsten verwendete Organismus zur Produktion niedermolekularer Substanzen und Proteine.

Grundsätzlich werden zwei unterschiedliche Mikroorganismen verwendet, die Mikroorganismen, die die Substanzen natürlicherweise produzieren und solche, die genetisch modifiziert wurden. Die zur genetischen Modifikation benötigten technischen Verfahren sind im Stand der Technik seit langem bekannt. Ziel ist es dabei die Gene, die für die Zielproteine bzw. zur Synthese der niedermolekularen Substanzen notwendig sind in die Wirtszelle einzubringen. Diese werden von der Wirtszelle transkribiert, translatiert, soweit nötig modifiziert und eventuell in das Medium ausgeschleust.

Die Wirtschaftlichkeit eines biotechnologischen Verfahrens hängt entscheidend von den erzielten Ausbeuten des Produktes ab. Diese können durch das Expressionssystem (Wirtszelle, genetische Elemente etc.), den Fermentationsparametern und den Nährmedien optimiert werden.

Grundlegend können die Wirtszellen in zweierlei Weise modifiziert werden. So kann die neue genetische Information ins Genom integriert werden (filamentöse Pilze, Hefen, etc.) und/oder auf einem extrachromosomalen Element (z.B. Plasmid) eingebracht werden (Prokaryonten, Hefen etc.). Bei der genetischen Integration der Gene ins Genom bleiben diese auch ohne Selektionsdruck gut in der Wirtszelle erhalten. Nachteilig ist aber, dass bei Prokaryonten nur eine Kopie des Gens im Wirt vorhanden ist und die Integration weiterer Kopien desselben Gens zur Steigerung der Produktbildung über den Gendosiseffekt, aufgrund von sequenzspezifischen Rekombinationsereignissen sehr anspruchsvoll ist (EP 0284126 B1).

Bei der Nutzung extrachromosomaler DNA wird in der Regel die genetische Information des Zielproteins in Form eines Plasmids in den *E*. *coli* Produktionsstamm transformiert. Da sich auch hier der Gendosis-Effekt auswirkt, wird eine möglichst hohe Anzahl an Plasmidkopien pro Zelle angestrebt. Da ein solches genetische Element aufgrund der Belastung, sowohl durch die Replikation des Plasmids als auch durch die Produktion des Zielproteins, leicht aus der Zelle verloren geht, muss über die gesamte Kultivierung ein Selektionsdruck ausgeübt werden. Standardmäßig werden als Selektionsmarker Antibiotika-Resistenzgene verwendet, die damit der Zelle, die ein solches Element aufweist, ein Wachstum in Gegenwart von Antibiotika ermöglicht. Entsprechend können sich nur die Zellen vermehren, die ein Plasmid tragen. Da durch den Plasmidverlust auch die Fähigkeit zur Produktion der Zielsubstanz bzw. des Zielproteins verloren geht, hat dies einen direkten Effekt auf die Ausbeuten, die in der Fermentation erzielt werden können.

Der Einsatz von Antibiotikaresistenzen als Selektionsmarker wird in den letzten Jahren zunehmend kritisch betrachtet. Zum einen ist der Einsatz von Antibiotika recht teuer, insbesondere, wenn die Resistenz auf einem Antibiotika abbauenden Enzym beruht, da das Antibiotikum stetig nachdosiert werden muss. Andererseits trägt der weitverbreitete Einsatz in der Medizin und anderen Gebieten zur Ausbreitung der Resistenzgene auf andere, teilweise pathogene Stämme bei. Dies hat negative Folgen für die Behandlung von Krankheiten.

Im Stand der Technik sind inzwischen auch antibiotikafreie Selektionssysteme entwickelt worden. Mehrere unterschiedliche antibiotikafreie Systeme wurden entwickelt. Diese können in drei unterschiedliche Grundsysteme unterteilt werden. Die Verwendung von Auxotrophien, Toxin-Antitoxin-Systemen und weiteren Verfahren.

In die Kategorie der weiteren Verfahren fallen Mechanismen, die keinem allgemeinen Prinzip folgen, z.B. Suppressor t-RNAs, fab I/Triclosan (FA-Synthese), Operator/Repressor Titration (Peubez et al. Microbial Cell Fac, 2010, 9:65). Diese Verfahren werden aber in der Regel zur Synthese von DNA und DNA-Fragmenten zur Gentherapie eingesetzt und sind nicht für die Produktion hoher Ausbeuten an Zielsubstanzen optimiert. Teilweise werden anstatt von Antibiotika andere Stoffe z. B. Triclosan, Herbizide und Schwermetalle, zur Selektion eingesetzt, die aber auch mit gesundheitlichen Bedenken behaftet sind. So werden beispielsweise von Herrero et al. (1990, J.Bacteriol. 172, 6557-6567) Resistenzgene gegen Herbizide und Schwermetalle als Selektionsmarker beschrieben.

Toxin-Antitoxin-Systeme (Hok-Sok, ccdA/B etc.) bestehen aus zwei genetischen Elementen, die sowohl beide auf dem Plasmid, als auch chromosomal und auf dem Plasmid kodiert sein können (Gerdes et al. Proc Natl Acad Sci USA, 1986, 83:3116-20). Das Antitoxin wirkt dabei neutralisierend auf das Toxin. Bei Verlust des Plasmids fehlt dieser Mechanismus und die plasmidfreie Zelle stirbt aufgrund des chromosomal kodierten bzw. langlebigen Toxins.

Eine weitere bekannte Methode zur Selektion ist die Komplementierung auxotropher Stämme. Hier werden Gene im Genom des Produktionsstammes entfernt bzw. inaktiviert, die essentielle Funktionen im Stoffwechsel haben. Entsprechend werden solche Gene als essentielle Gene bezeichnet. Die somit entstandenen auxotrophen Stämme können nur dann wachsen, sich vermehren oder überleben, wenn die Stoffwechselfunktion umgangen oder wieder hergestellt wird. Dies kann sowohl durch Fütterung entsprechender Vorstufen oder Endprodukte des Stoffwechsels (Aminosäuren, Basen etc.) oder durch Einbringen des Gens, das im Wirtsgenom deaktiviert wurde, erreicht werden. Das Patent EP 0284126 B1 nennt Auxotrophiemarker des Aminosäurestoffwechsels. Da Auxotrophien leicht durch Zugabe des notwendigen Stoffwechselproduktes ins Medium supplementiert werden können, lassen sich diese Stämme einfach erzeugen. Denn die Zellen können in Gegenwart des Stoffwechselprodukts auch ohne Plasmid kultiviert werden. Durch Transformation wird die Information zur Synthese der Aminosäure/Base wieder in die Zelle eingeführt und die Zelle kann auch ohne Supplementation wachsen. Entsprechend muss die Selektion auf Minimalmedien ohne das Supplement erfolgen.

In der Praxis konnte sich der Einsatz solcher Auxotrophien als Selektionsmarker bisher nicht durchsetzen, da in der industriellen Fermentation üblicherweise Medien mit komplexer Zusammensetzung verwendet werden. Hier sind in der Regel aus Kostengründen Abfallstoffe Bestandteil des Fermentationsmediums, wie z.B. Rückstände von Getreide (Ethanolherstellung), Mais (Stärkeherstellung), Kartoffeln (Stärkegewinnung) oder Hefeextrakt. Diese dienen sowohl als Kohlenstoff- als auch als Stickstoffquellen. Teilweise sind diese Bestandteile nicht genau definiert, enthalten aber Aminosäuren, Basen, Vitamine etc., die aus dem Medium aufgenommen werden können. In der industriellen Fermentation ist es daher schwer, wenn nicht unmöglich, einen ausreichenden Selektionsdruck mit auxotrophen Stämmen aufzubauen.

Auch die Verwendung einer Auxotrophie im Glukosestoffwechsel, wie sie in WO 2008/135113 A1 beschrieben wird, hat in den industriell verwendeten Medien keine ausreichende Selektivität. Zwar können die Mikroorganismen besonders gut auf Glucose wachsen und sich schneller vermehren, als dies in Medien mit anderen C-Quellen der Fall ist, aber wegen der höheren Belastung durch das Plasmid bzw. die Produktion der Zielsubstanz wird dieser Vorteil wieder aufgehoben. Andere C-Quellen stehen in komplexen Medien zur Verfügung und werden von den Zellen verwendet.

Dies gilt auch für die in WO 07039632 A1 beschriebene Verwendung des pyrC-Gens (Dihydroorotase) als Auxotrophiemarker. Dieses Enzym befindet sich am Anfang der Pyrimindin-Basen Synthese und die Inaktivierung führt auch zur Inhibition der Denovo Synthese. Jedoch können die Basen aus dem Medium aufgenommen und verwertet werden und ein entsprechender Selektionsdruck kann in industriellen Medien nicht aufgebaut werden.

Ausnahmen stellen Auxotrophien für das essentielle Thymidin und D-Alanin dar, die auch in komplexen Bestandteilen lediglich in Spuren oder gar nicht in den Fermentationsmedien vorkommen (EP 0251579 A1; EP 0185512 B1). Aber auch diese Systeme sind nicht geeignet für die effiziente Produktion im Hochzelldichte-Verfahren, das in der Regel angestrebt wird. Da bei diesem Verfahren die Zellen teilweise absterben und lysieren, werden entsprechend Thymidin und D-Alanin bzw. andere Aminosäuren etc. freigesetzt, die wiederum die Auxotrophien supplementieren können.

Insgesamt muss festgestellt werden, dass trotz jahrelanger Erfahrung zur fermentativen Produktion von niedermolekularen Substanzen und Proteinen bisher kein universell einsetzbares System entwickelt wurde, außer jenem über teure oder gesundheitlich bzw. ökologisch bedenkliche Substanzen, wie Antibiotika. Auch die verschiedenen Ansätze zur Selektion über Auxotrophiemarker haben wegen der in der Industrie verwendeten komplexen Wachstumsmedien bisher nur zu dürftigen Ergebnissen geführt.

Dies gilt prinzipiell für Mikroorganismen, aber insbesondere für weniger robuste Produktionsstämme, wie z.B. leaky Stämme, die aufgrund ihrer speziellen Eigenschaften (Freisetzung von Proteinen ins Medium) genutzt werden. Bei der industriellen Nutzung dieser Stämme im technischen Maßstab werden üblicherweise komplexe Bestandteile im Medium verwendet.

Die Nutzung definierter Medien aus gereinigten Bestandteilen ist für die Herstellung von industriell genutzten Produkten aus Kostengründen nicht wirtschaftlich.

Aufgabe der Erfindung ist es, einen Mikroorganismenstamm zur Produktion von niedermolekularen Substanzen oder Proteinen bereitzustellen, der auch in Medien mit Komplexbestandteilen stabil bleibt und bei dem das Produktionsplasmid nicht durch ein Antibiotikum/Resistenzmarker-System in der Zelle stabilisiert wird.

Diese Aufgabe wird gelöst durch einen Mikroorganismenstamm enthaltend in seinem Genom eine Mutation in einem Gen, welche eine Auxotrophie des Stammes bewirkt, sowie ein Produktionsplasmid kodierend mindestens ein Enzym zur Produktion einer niedermolekularen Substanz oder mindestens ein rekombinantes Protein sowie eine funktionelle Kopie des Gens, dessen chromosomale Inaktivierung die Auxotrophie bewirkt, dadurch gekennzeichnet, dass es sich bei der Auxotrophie um eine nicht fütterbare Auxotrophie handelt und es sich um das pyrH-Gen oder dessen homologe Gene handelt.

Im Rahmen der Erfindung ist unter einer nicht fütterbaren Auxotrophie zu verstehen, dass die Auxotrophie das Wachstum der Mikroorganismenzelle vermindert oder den Tod der Mikroorganismenzelle bewirkt und nicht durch Zugabe von stoffwechselspezifischen Vor-, Zwischen- und/oder Endprodukten in das Wachstumsmedium supplementierbar ist.

Ein vermindertes Wachstum liegt im Sinne der Erfindung vor, wenn die Wachstumsrate des Stammes in einer Fermentation nach der Mutation des Gens im Vergleich zu der Wachstumsrate des Stammes vor der Mutation des Gens auf ≤ 10% erniedrigt ist, bevorzugt wenn kein Wachstum mehr stattfindet.

Besonders bevorzugt führt die Mutation zur Inaktivierung eines Gens, bewirkt so eine nicht fütterbaren Auxotrophie und, damit den Tod der Mikroorganismenzelle.

Im Genom einer Zelle des erfindungsgemäßen Mikroorganismenstammes ist demnach ein Gen inaktiviert, welches essentiell für einen für das Wachstum oder Überleben der Zelle notwendigen anabolen Stoffwechselweg ist, wobei Wachstum oder Überleben der Zelle nicht durch Zugabe von stoffwechselspezifischen Vor-, Zwischen- und/oder Endprodukten in das Wachstumsmedium wieder erreicht werden kann.

Ein nicht fütterbares Auxotrophie-Gen im Sinne der Erfindung ist ein Gen im Genom einer Mikroorganismenzelle, dessen Inaktivierung nicht durch Zugabe von stoffwechselspezifischen Vor-, Zwischen- und/oder Endprodukten in das Wachstumsmedium komplementiert werden kann und dessen Inaktivierung zu einer verminderten Wachstumsrate oder zum Tod der Mikroorganismenzelle führt.

Vorzugsweise führt die Mutation des Gens, welche die nicht fütterbare Auxotrophie des Stammes bewirkt, zur Inaktivierung dieses Gens oder zur Inaktivierung der Aktivität des durch das Gen kodierten Genprodukts.

Beispiele für nicht fütterbare Auxotrophie-Gene (lethale Gene) sind in Baba et al. (2006, Mol. Syst. Biol. 2:2006.0008) beschrieben. Erfindungsgemäß handelt es sich um pyrH oder dessen homologe Gene.

Erfindungsgemäß handelt es sich um das pyrH-Gen oder homologe Gene mit gleicher Funktion bzw. Aktivität.

Ein Gen im Sinn der vorliegenden Erfindung umfasst neben dem DNA Abschnitt der transkribiert wird auch die DNA Abschnitte, die an der Regulation dieses Kopiervorgangs beteiligt sind, also die regulatorischen Elemente des Gens, wie bevorzugt Promotoren und Terminatoren.

Unter homologen Genen sind vorzugsweise Gene zu verstehen, die für ein Protein mit der gleichen Aktivität kodieren wie das durch das genannte Gen kodierte Protein und eine Sequenzidentität größer 30%, besonders bevorzugt größer 70%, zu den jeweils aus Datenbanken bekannten Sequenzen der genannten Gene im jeweiligen Mikroorganismus aufweisen.

Das pyrH-Gen kodiert für das Enzym UMP-Kinase (EC: 2.7.4.22; UK, Uridinmonophosphat Kinase, Uridylatkinase, Uridin 5'-Monophosphat (UMP) Kinase). Dieses Enzym aktiviert UMP zu UDP unter Verwendung von ATP. UDP wird in einem nächsten Schritt von einer weiteren Kinase (UDP-Kinase) zu UTP aktiviert, dass sowohl zur Synthese von RNA also auch zur Synthese von CTP (Cytosintriphosphat) und TTP (Thymidintriphoshpat) verwendet werden kann. CTP und TTP sind wiederum Bestandteil von RNA (CTP) und DNA (dCTP und dTTP). UMP ist damit der Ursprung für Pyrimidinbausteine von RNA und DNA. Da eine Umwandlung von Uracil in Cytosin oder Thymidin erst auf der Ebene des Triphosphats erfolgt führt eine Deaktivierung der UMP-Kinase zu einem völligen Block dieses Syntheseweges.

Grundsätzlich kann die Zelle nur die Monophosphate der drei Basen aus dem Medium aufnehmen. Für die Umwandlung des UMP zu UTP ist die UMP-Kinase essentiell. Damit ist diese Auxotrophie nicht durch Fütterung supplementierbar und somit unabhängig von der Medienzusammensetzung.

Das *pyrH-*Gen ist charakterisiert durch SEQ ID No. 1. Das *pyrH-*Genprodukt (PyrH) ist charakterisiert durch SEQ ID No. 2.

Im Rahmen der vorliegenden Erfindung sind *pyrH*-Homologe Gene, die für ein Protein mit PyrH Aktivität kodieren und die eine Sequenzidentität größer 30% zu SEQ ID No. 1 aufweisen. Besonders bevorzugt ist eine Sequenzidentität größer 70% zu SEQ ID No. 1. Insbesondere bevorzugt handelt es sich um das *pyrH*-Gen.

PyrH-Homologe sind Proteine mit einer Sequenzidentität größer 30% zu SEQ ID No. 2, die eine UMP-Kinase-Aktivität gemäß EC-Nummer 2.7.4.22 aufweisen. Besonders bevorzugt weisen PyrH-Homologe eine UMP-Kinase-Aktivität gemäß EC-Nummer 2.7.4.22 und eine Sequenzidentität größer 70% zu SEQ ID No. 2 auf. Insbesondere bevorzugt handelt es sich um das PyrH Protein.

Die PyrH Aktivität in einer Zelle kann entsprechend dem von Bucurenci et al. (1998, J. Bact. 180: 473-77) beschriebenen Test bestimmt werden.

Der Grad der DNA-Identität wird durch das Programm "nucleotide blast", zu finden auf der Seite http://blast.ncbi.nlm.nih.gov/, bestimmt, welches auf dem blastn-Algorithmus basiert. Als Algorithmus-Parameter für ein Alignment zweier oder mehrerer Nukleotidsequenzen wurden die voreingestellten Parameter genutzt. Die voreingestellten generellen Parameter sind: Max target sequences = 100; Short queries = "Automatically adjust parameters for short input sequences"; Expect Threshold = 10; Word size = 28; Automatically adjust parameters for short input sequences = 0. Die entsprechenden voreingestellten Scoring Parameter sind: Match/Mismatch Scores = 1,-2; Gap Costs = Linear.

Für den Vergleich von Proteinsequenzen wird das Programm "protein blast", auf der Seite http://blast.ncbi.nlm.nih.gov/, genutzt. Dieses Programm greift auf den blastp-Algorithmus zurück. Als Algorithmus-Parameter für ein Alignment zweier oder mehrerer Proteinsequenzen wurden die voreingestellten Parameter genutzt. Die voreingestellten generellen Parameter sind: Max target sequences = 100; Short queries = "Automatically adjust parameters for short input sequences"; Expect Threshold = 10; Word size = 3; Automatically adjust parameters for short input sequences = 0. Die voreingestellten Scoring Parameter sind: Matrix = BLOSUM62; Gap Costs = Existence: 11 Extension: 1; Compositional adjustments = Conditional compositional score matrix adjustment.

Zur Herstellung eines erfindungsgemäßen Stamms wird in einen geeigneten Mikroorganismenstamm ein temperatursensitives Plasmid eingebracht, welches eine funktionelle Kopie eines nicht fütterbaren Auxotrophie-Gens, das mutiert oder deletiert werden soll, besitzt. Anschließend wird das entsprechende nicht fütterbare Auxotrophie-Gen im Genom des Stammes inaktiviert. Danach wird in diesem Stamm das temperatursensitive Plasmid bei nicht-permissiver Temperatur gegen ein Produktionsplasmid ausgetauscht, wobei das Produktionsplasmid kodierend mindestens ein Enzym zur Produktion einer niedermolekularen Substanz oder mindestens ein rekombinantes Protein auch eine funktionelle Kopie des nicht fütterbaren Auxotrophie-Gens enthält.

Durch dieses Verfahren wird sichergestellt, dass das Produktionsplasmid während eines Fermentationsprozesses zur Herstellung niedermolekularer Verbindungen oder von rekombinanten Proteinen auch in komplexen Medien stabil in der Zelle erhalten bleibt. Somit können erfindungsgemäße Stämme plasmidverlustfrei in Abwesenheit eines zugesetzten selektiven Agens bzw. eines Auxotrophie kompensierenden Additivs kultiviert werden.

Als Ausgangsstamm zur Herstellung eines erfindungsgemäßen Stammes ist grundsätzlich jeder Mikroorganismenstamm geeignet, der ein Gen besitzt, dessen Inaktivierung zu einer nicht-fütterbaren Auxotrophie des Stamms führt.

Bevorzugt handelt es sich bei dem Ausgangsstamm zur Erzeugung eines erfindungsgemäßen Stammes um einen Enterobacteriaceen-Stamm, insbesondere bevorzugt um einen Stamm der Spezies E*scherichia coli.*

Bei den *E. coli* Stämmen sind solche bevorzugt, die eine "leaky"-Mutation aufweisen. Unter einer "Leaky-Mutation" ist eine Mutation in einem Gen für ein Stukturelement der äußeren Zellmembran oder der Zellwand, ausgewählt aus der Gruppe der omp-Gene, tol-Gene, excD-Gen, excC-Gen, lpp-Gen, pal-Gen, env-Gene und lky-Gene zu verstehen, die dazu führt, dass die Zellen vermehrt periplasmatische Proteine ins Medium abgeben (Shokri et al., Appl. Microbiol. Biotechnol. 60 (2003), 654-664). Vorzugsweise handelt es sich um eine "leaky"-Mutation im lpp-Gen, besonders bevorzugt um eine Mutation ausgewählt aus der Gruppe lpp1-Mutation, lpp-Deletionsmutation und Mutation des Glycin-Rests an Position 14 des Lpp-Proteins (Zählweise inkl. Signalpeptid), wie z.B. die lpp-3-Mutation. Eine lpp1-Mutation ist eine Mutation im lpp-Gen, die zu einem Austausch des Arginin-Rests an Position 77 gegen einen Cystein-Rest führt, eine lpp3-Mutation ist eine Mutation im lpp-Gen, die zu einem Austausch des Glycin-Rests an Position 14 gegen einen Asparaginsäure-Rest führt. Diese Mutationen sind in US2008254511 detailliert beschrieben. Bei der lpp-Deletionsmutation handelt es sich vorzugsweise um eine Deletion von mindestens einem Nukleotid im lpp-Gen selbst oder in der Promotorregion des lpp-Gens, welche dazu führt, dass die Zellen eine erhöhte Leakiness für periplasmatische Proteine aufweisen.

Unter erhöhter Leakiness ist im Sinne der vorliegenden Erfindung zu verstehen, dass nach einer Fermentation der Zellen eine höhere Konzentration von periplasmatischen Proteinen, z. B. der Alkalischen Phosphatase, im Nährmedium vorliegt, als bei einer Fermentation des E. *coli*-Stammes W3110 (ATCC 27325) unter den gleichen Bedingungen.

Verfahren zur Inaktivierung eines Gens in einem Mikroorganismenstamm sind im Stand der Technik bekannt. Sie sind im Folgenden detailliert für die Mutation des pyrH-Gens sowie dessen Genprodukte beschrieben. Analog sind diese Verfahren auch für andere Gene, deren Inaktivierung eine nicht fütterbare Auxotrophie eines Mikroorganismenstammes bewirkt, anwendbar. Das temperatursensitive Plasmid enthält einen temperatursensitiven Replikationsursprung und eine funktionelle Kopie des nicht fütterbaren Auxotrophie-Gens. Außerdem enthält dieses Plasmid ein Selektionsmarker-Gen für die Selektion von Transformanten. Bei dem Selektionsmarker handelt es sich beispielsweise um eine Antibiotikaresistenz.

Ein bevorzugtes Beispiel für einen temperatursensitiven Replikationsursprung ist "oriR101 & repA101-ts", ein Abkömmling des Replikationsursprunges von Plasmid pSC101 (Hashimoto-Gotoh et al.; Gene, 2000, 241:1:185-191), der u.a. auf den Plasmiden pKD20 und pKD46 (Datsenko und Wanner, 2000, P.N.A.S. 97: 6640-6645) lokalisiert ist.

Das temperatursensitive Plasmid wird mit einer dem Fachmann bekannten Transformationstechnik, z.B. TSS-, CaCl/RbCl-, Elektroporations-Methode, in die Zelle eingebracht.

Auf Zellen, die das temperatursensitive Plasmid beinhalten, wird mittels des Selektionsmarkers, der auf dem temperatursensitiven Plasmid vorhanden ist, selektiert, während die Zellen bei für das Plasmid permissiver Temperatur inkubiert werden.

Ein derartiges temperatursensitives Plasmid lässt sich gegen das Produktionsplasmid austauschen, indem der Mikroorganismenstamm nach der Transformation mit dem Produktionsplasmid bei einer für das temperatursensitive Plasmid nicht-permissiven Temperatur kultiviert wird. Ein bevorzugter nicht-permissiver Temperaturbereich ist 37-45°C, besonders bevorzugt 39-43°C.

Das Produktionsplasmid wird mit einer dem Fachmann bekannten Transformationstechnik, z.B. TSS-, CaCl/RbCl-, Elektroporations-Methode, in die Zelle eingebracht.

Die Kultivierung der Transformanten kann sowohl auf Agar-Platten wie in Flüssigkultur erfolgen. In einer bevorzugten Ausführungsform wird der Mikroorganismenstamm unmittelbar nach der Transformation mit dem Produktionsplasmid für 30-90 min, bevorzugt 60 min, einem Temperaturschock bei 47-55°C, bevorzugt bei 52°C, ausgesetzt. Anschließend erfolgt die weitere Inkubation bei der oben genannten nicht permissiven Temperatur. Dadurch wird in einem Schritt das temperatursensitive Plasmid gegen das Produktionsplasmid ausgetauscht und ein erfindungsgemäßer Produktionsstamm zur antibiotikafreien Produktion von niedermolekularen Substanzen oder rekombinanten Proteinen generiert.

Als Produktionsplasmid wird beispielsweise einer der folgenden bekannten Expressionsvektoren verwendet: pJF118EH, pKK223-3, pUC18, pBR322, pACYC184, pASK-IBA3 oder pET.

Das Produktionsplasmid enthält neben der funktionellen Kopie des nicht fütterbaren Auxotrophie-Gens ein oder mehrere Zielgene sowie die zur Expression dieser Zielgene notwendigen Expressionssignale, wie z.B. Promotor-, Operator-, und Terminatorsequenzen. Die Zielgene kodieren für mindestens ein Enzym zur Produktion einer niedermolekularen Substanz oder mindestens ein rekombinantes Protein.

Bei den niedermolekularen Substanzen handelt es sich um Grundbausteine (Basen, Aminosäuren, Fettsäuren etc.) sowie Sekundärmetabolite (Vitamine, Antioxidantien etc.) die der Organismus synthetisieren kann. Bevorzugt sind Aminosäuren, besonders bevorzugt die Aminosäure L-Cystein und davon abgeleitete Verbindungen.

Bei den rekombinanten Proteinen handelt es sich vorzugsweise um heterologe Proteine.

Unter einem heterologen Protein ist ein Protein zu verstehen, das nicht zum Proteom, d.h. der gesamten natürlichen Protein-Ausstattung, des Wirtsorganismus gehört.

Bevorzugt handelt es sich bei dem heterologen Protein um ein eukaryotisches Protein, besonders bevorzugt um ein Protein, welches eine oder mehrere Disulfid-Brücken enthält oder welches in seiner funktionellen Form als Di- oder Multimer vorliegt, d.h. dass das Protein eine Quartärstruktur besitzt und aus mehreren identischen (homologen) oder nicht-identischen (heterologen) Untereinheiten aufgebaut ist.

Eine bevorzugte Klasse von Proteinen, die aus mehreren Proteinuntereinheiten besteht, sind Antikörper oder Fragmente von Antikörpern. Besonders bevorzugt sind funktionelle Fab-Antikörperfragmente.

Im Folgenden wird die Herstellung eines erfindungsgemäßen Mikroorganismenstammes beschrieben, bei dem das nicht fütterbare Auxotrophie-Gen pyrH mutiert ist.

Als Ausgangsstämme für die Herstellung eines erfindungsgemäßen Mikroorganismenstammes mit einer genomischen pyrH Inaktvierung ist grundsätzlich jeder Wirtsorganismus geeignet, der ein Gen für die UMP-Kinase PyrH besitzt.

Verfahren zur Inaktivierung des pyrH-Gens in einem Mikroorganismenstamm sind im Stand der Technik bekannt. Das pyrH-Gen kann beispielsweise dadurch inaktiviert werden, dass eine Mutation (Substitution, Insertion oder Deletion einzelner oder mehrerer Nukleotide) in den Leserahmen des *pyrH-*Gens eingebracht wird, welche dazu führt, dass die spezifische Aktivität von PyrH inaktiviert wird. Dem Fachmann sind Verfahren zur Erzeugung solcher *pyrH*-Allele bekannt. So kann beispielsweise mittels des in Link et al. (1997, J. Bacteriol. 179: 6228-37) beschriebenen Verfahrens die Einführung chromosomaler Mutationen in ein Gen über den Mechanismus der homologen Rekombination erfolgen. Die chromosomale Deletion des gesamten *pyrH-*Gens oder eines Teils davon ist beispielsweise mit Hilfe des λ-Red Rekombinase-Systems nach der von Datsenko und Wanner (2000, Proc. Natl. Acad. Sci. USA. 97: 6640-5) beschriebenen Methode möglich. *pyrH*-Allele können auch über eine Transduktion mittels P1-Phagen oder Konjugation aus einem Stamm mit *pyrH*-Mutation auf einen *pyrH*-Wildtyp-Stamm übertragen werden, wobei das *pyrH*-Wildtyp-Gen im Chromosom gegen das entsprechende *pyrH*-Allel ersetzt wird.

Darüber hinaus kann das pyrH-Gen einer Zelle auch dadurch inaktiviert werden, dass mindestens ein für die Expressionsregulation notwendiges Element (z.B. Promotor, Enhancer, Riboso men-Bindestelle) durch Substitution, Insertion oder Deletion einzelner oder mehrerer Nukleotide mutiert wird. Eine Inaktivierung im Sinne der Erfindung liegt vor, wenn die Wachstumsrate der Zellen in einer Fermentation durch die Inaktivierung des Gens im Vergleich zu den Zellen vor der Mutation auf ≤ 10% erniedrigt ist, bevorzugt wenn kein Wachstum mehr stattfindet. Besonders bevorzugt führt die Mutation zum Tod der Mikroorganismenzelle.

Da die Inaktivierung des pyrH-Gens zu einer nicht-fütterbaren Auxotrophie führt, muss eine funktionelle Kopie des pyrH-Gens bereits vor der chromosomalen Inaktivierung in der Zelle vorhanden sein. Dies kann durch das transiente Einbringen eines temperatursensitiven Plasmids, das eine funktionelle Kopie des pyrH-Gens enthält, erreicht werden.

In solchen Zellen, die das temperatursensitive Plasmid enthalten, kann nun durch die genannten Methoden das pyrH-Gen im Genom inaktiviert werden.

In einem weiteren Schritt wird dieses temperatursensitive Plasmid gegen das Produktionsplasmid, das auch eine funktionelle Kopie des pyrH-Gens enthält, ausgetauscht. Dies geschieht vorzugsweise in der bereits beschriebenen Art und Weise.

Bei der Herstellung von rekombinanten Proteinen unterscheidet man zwischen einer cytoplasmatischen und einer sekretorischen Produktion. Während bei der cytoplasmatischen Produktion das Zielprotein im Cytoplasma der Zelle angehäuft wird, wird bei der sekretorischen Produktion das Zielprotein in das Periplasma bzw. in das Kulturmedium transloziert. Im Rahmen der Erfindung wird die sekretorische Produktion bevorzugt. Insbesondere bevorzugt ist die sekretorische Produktion des Zielproteins in das Kulturmedium.

Für die sekretorische Produktion von Proteinen, d.h. für die Translokation des Proteins aus dem Cytoplasma in das Periplasma bzw. das Kulturmedium, ist es notwendig, das 5'-Ende des Gens des zu produzierenden Proteins *in frame* mit dem 3'-Ende einer Signalsequenz für den Protein-Export zu verknüpfen. Dazu sind prinzipiell die Gene aller Signalsequenzen geeignet, die in *E*. *coli* zu einer Translokation des Zielproteins in das Periplasma führen. In *E*. *coli* sind drei Haupttranslokationswege bekannt: der SEC-, TAT- und SRP-Weg. Bevorzugt sind solche Signalsequenzen, die eine Translokation über den SEC-Apparat ermöglichen. Verschiedene derartige Signalsequenzen sind im Stand der Technik beschrieben, so z.B. die Signalsequenzen der folgenden Gene: phoA, ompA, pelB, ompF, ompT, lamB, malE, dsbA, Staphylococcal protein A, StII und andere (Choi and Lee, Appl. Microbiol. Biotechnol. 64 (2004), 625-635) .

Erfindungsgemäß bevorzugt ist die Signalsequenz des phoA- oder des ompA-Gens von *E. coli* oder die Signalsequenz für eine Cyclodextrin-Glycosyltransferase (CGTase) aus *Klebsiella pneumoniae* M5a1, oder die von dieser Signalsequenz abgeleitete Sequenz, die in US2008076157 offenbart ist.

Die Kultivierung (Fermentation) der Zellen, die ein Produktionsplasmid enthalten, erfolgt nach üblichen, dem Fachmann bekannten Fermentationsverfahren in einem Bioreaktor (Fermenter) ohne Zugabe von Antibiotika.

Die Erfindung betrifft auch ein Verfahren zur Herstellung von niedermolekularen Substanzen oder rekombinanten Proteinen mittels eines Mikroorganismenstamms, das dadurch gekennzeichnet ist, dass ein erfindungsgemäßer Mikroorganismenstamm eingesetzt wird und ein antibiotikafreies Fermentationsmedium verwendet wird.

Die Fermentation findet in einem üblichen Bioreaktor, beispielsweise einem Rührkessel, einem Blasensäulen-Fermenter oder einem Airlift-Fermenter statt. Bevorzugt ist ein Rührkessel-Fermenter im technischem Maßstab und damit mit einer Größe von >100 1.

Bei der Fermentation werden die Zellen des erfindungsgemäßen Stammes in einem Flüssigmedium kultiviert, wobei verschiedene Parameter wie z.B. die Nährstoffzufuhr, der Sauerstoff-Partialdruck, der pH-Wert und die Temperatur der Kultur laufend kontrolliert und genau gesteuert werden. Der Zeitraum der Kultivierung beträgt vorzugsweise 16-150 h, besonders bevorzugt 24-72 h.

Als Fermentationsmedien kommen alle gängigen, dem Fachmann bekannten Medien für die Kultivierung von Mikroorganismen in Frage. Diese Fermentationsmedien sind jedoch frei von einem Antibiotikum.

Es können Komplexmedien oder Minimalsalzmedien, denen ein definierter Anteil von Komplex-Komponenten wie z.B. Pepton, Trypton, Hefeextrakt, Melasse oder Corn Steep Liquor zugesetzt wird, verwendet werden. Bevorzugt wird ein Medium mit Komplexmedienkomponenten.

Als primäre Kohlenstoffquelle für die Fermentation können alle von den Zellen verwertbaren Zucker, Zuckeralkohole oder organische Säuren bzw. deren Salze verwendet werden. Dabei werden bevorzugt Glucose, Laktose oder Glycerin eingesetzt. Besonders bevorzugt sind Glucose und Laktose. Auch ist eine kombinierte Fütterung mehrerer verschiedener Kohlenstoffquellen möglich. Die Kohlenstoffquelle kann dabei zu Beginn der Fermentation vollständig im Fermentationsmedium vorgelegt werden oder es wird nichts oder nur ein Teil der Kohlenstoffquelle zu Beginn vorgelegt und die Kohlenstoffquelle wird über den Verlauf der Fermentation zugefüttert. Besonders bevorzugt ist dabei eine Ausführungsform, bei der ein Teil der Kohlenstoffquelle vorgelegt wird und ein Teil gefüttert wird. Besonders bevorzugt wird die Kohlenstoffquelle in einer Konzentration von 10-30 g/l vorgelegt, die Fütterung gestartet, wenn die Konzentration auf kleiner 5 g/l abgefallen ist und so gestaltet, dass die Konzentration unter 5 g/l gehalten wird.

Der Sauerstoff-Partialdruck (pO₂) in der Kultur beträgt vorzugsweise zwischen 10 und 70 % Sättigung. Bevorzugt wird ein pO₂ zwischen 20 und 60 %, besonders bevorzugt liegt der pO₂ zwischen 45 und 55 % Sättigung.

Der pH-Wert der Kultur liegt vorzugsweise zwischen pH 6 und pH 8. Bevorzugt wird ein pH-Wert zwischen 6,5 und 7,5, besonders bevorzugt liegt der pH-Wert der Kultur zwischen 6,8 und 7,2.

Die Temperatur der Kultur liegt zwischen 15 und 45°C. Bevorzugt ist ein Temperaturbereich zwischen 18 und 40°C, besonders bevorzugt ist ein Temperaturbereich zwischen 25 und 35 °C, ganz besonders bevorzugt sind 30°C.

Bei einer bevorzugten Ausrichtung handelt es sich bei der Fermentation um eine Hochzelldichte-Fermentation. Unter einer Hochzelldichte-Fermentation ist eine Fermentation zu verstehen, in deren Verlauf Zelltrockengewichte von mehr als 50 g/l erreicht werden. Besonders bevorzugt werden Zelltrockengewichte von mehr als 70 g/l.
Fig. 1 zeigt eine Restriktions- und Funktionskarte von Plasmid pKD46 aus Bsp. 2.
Fig. 2 zeigt eine Restriktions- und Funktionskarte des Plasmids pAF-ts-pyrH hergestellt in Bsp. 2.
Fig. 3 zeigt eine Restriktions- und Funktionskarte des Plasmids pAF-ts-plsC hergestellt in Bsp. 2.
Fig. 4 zeigt eine Restriktions- und Funktionskarte das Plasmids pMT1 verwendet in Bsp. 5.
Fig. 5 zeigt eine Restriktions- und Funktionskarte des Expressionsplasmids pcysEX-GAPDH-ORF306_tetR hergestellt in Bsp. 5.
Fig. 6 zeigt eine Restriktions- und Funktionskarte des Expressionsplasmids pCGT_tetR hergestellt in Bsp. 6.
Fig. 7 zeigt eine Restriktions- und Funktionskarte des Expressionsplasmids pFab-anti-Lysozym_tetR hergestellt in Bsp. 7.
Fig. 8 zeigt eine Restriktions- und Funktionskarte des Expressionsplasmids pcysEX-GAPDH-ORF306_pyrH1_tetR hergestellt in Bsp. 8.
Fig. 9 zeigt eine Restriktions- und Funktionskarte des Expressionsplasmids pCGT_pyrH1_tetR hergestellt in Bsp. 8.
Fig. 10 zeigt eine Restriktions- und Funktionskarte des Expressionsplasmids pFab-anti-Lysozyme_pyrH1_tetR hergestellt in Bsp. 8.
Fig. 11 zeigt eine Restriktions- und Funktionskarte des Expressionsplasmids pcysEX-GAPDH-ORF306_plsC1_tetR hergestellt in Bsp. 8.
Fig. 12 zeigt eine Restriktions- und Funktionskarte des Expressionsplasmids pCGT_plsC1_tetR hergestellt in Bsp. 8.
Fig. 13 zeigt eine Restriktions- und Funktionskarte des Expressionsplasmids pFab-anti-Lysozyme_plsC1_tetR hergestellt in Bsp. 8.
Fig. 14 zeigt eine Restriktions- und Funktionskarte des erfindungsgemäßen Produktionsplasmids pcysEX-GAPDH-ORF306_pyrH hergestellt in Bsp. 9.
Fig. 15 zeigt eine Restriktions- und Funktionskarte des erfindungsgemäßen Produktionsplasmids pCGT_pyrH hergestellt in Bsp. 9.
Fig. 16 zeigt eine Restriktions- und Funktionskarte des erfindungsgemäßen Produktionsplasmids pFab-anti-Lysozym_pyrH hergestellt in Bsp. 9.
Fig. 17 zeigt eine Restriktions- und Funktionskarte des erfindungsgemäßen Produktionsplasmids pcysEX-GAPDH-ORF306_plsC hergestellt in Bsp. 9.
Fig. 18 zeigt eine Restriktions- und Funktionskarte des erfindungsgemäßen Produktionsplasmids pCGT_plsC hergestellt in Bsp. 9.
Fig. 19 zeigt eine Restriktions- und Funktionskarte des erfindungsgemäßen Produktionsplasmids pFab-anti-Lysozym_plsC hergestellt in Bsp. 9.

Die folgenden Beispiele dienen der weiteren Erläuterung der Erfindung. Sämtliche eingesetzten molekularbiologischen und mikrobiologischen Verfahren wie Polymerase-Ketten-Reaktion (PCR), Gensynthese, Isolierung und Reinigung von DNA, Modifikation von DNA durch Restriktionsenzyme, Klenow-Fragment und Ligase, Transformation, P1-Transduktion etc., wurden in der dem Fachmann bekannten, in der Literatur beschriebenen oder von den jeweiligen Herstellern empfohlenen Art und Weise durchgeführt.

Die folgenden Beispiele, sofern sie sich auf das plsC-Gen beziehen, entsprechen nicht der Erfindung und dienen nur der Veranschaulichung.

### Beispiel 1: Amplifikation des Marker-Gens (pyrH- oder plsC) mit eigenem Promotor

Ein etwa 1,0 kb großes DNA-Fragment, welches für das *pyrH-*Gen inklusive nativer Promotoregion kodiert, wurde mit den Primern pyrH-NcoI-fw (SEQ ID No. 5) und pyrH-NcoI-rev (SEQ ID No. 6) amplifiziert. Als Matrize für die PCR-Reaktion diente chromosomale DNA des *E. coli* Stammes W3110 (ATCC 27325).

Das etwa 1,0 kb große PCR-Fragment wurde über eine Agarose-Gelelektrophorese gereinigt und mit dem "QIAquick Gel Extraction Kit" (Qiagen GmbH, Hilden, D) nach Angaben des Herstellers aus dem Agarosegel isoliert. Anschließend wurde das gereinigte PCR-Fragment mit dem Restriktionsenzym NcoI verdaut und bei -20°C gelagert. Analog wurde ein etwa 1,0 kb großes DNA-Fragment, welches für das *plsC*-Gen inklusive nativer Promotorregion kodiert, amplifiziert, gereinigt, verdaut und gelagert. Für die Amplifikation wurden die Primer plsC-NcoI-fw (SEQ ID No. 7) und plsC-NcoI-rev (SEQ ID No. 8) verwendet.

### Beispiel 2: Generierung der Plasmide pAF-ts-pyrH und pAF-ts-plsC mit temperatursensitivem Replikationsursprung

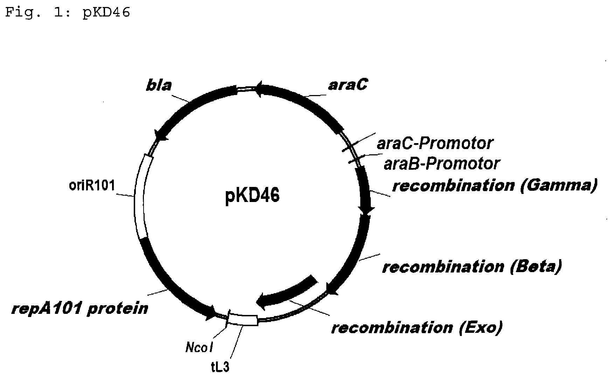

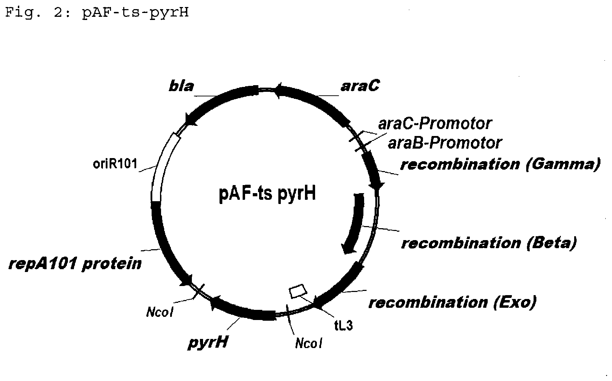

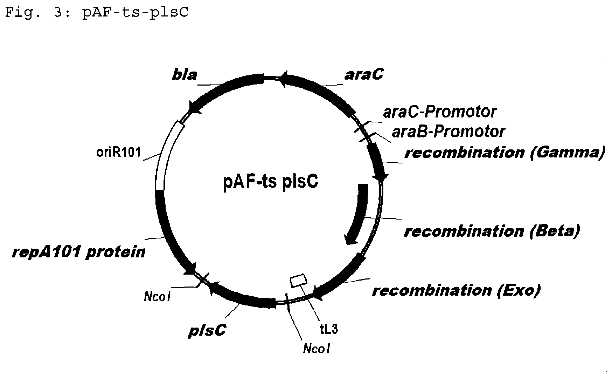

Als Ausgangsplasmid für die Konstruktion der Plasmide pAF-ts-pyrH und pAF-ts-plsC mit temperatur-sensitivem Replikationsursprung wurde das Plasmid pKD46 verwendet (Datsenko und Wanner, 2000, P.N.A.S. 97: 6640-6645). Eine Restriktions- und Funktionskarte von Plasmid pKD46 ist in Fig. 1 gezeigt. In die NcoI-Schnittstelle von pKD46 wurden die in Beispiel 1 beschriebenen, und mit NcoI verdauten PCR-Produkte kloniert, welche für das *pyrH*-Gen oder *plsC*-Gen mit eigenem Promotor kodieren. Die Ligationsansätze wurden in "DH5α™-T1R *E. coli-*Zellen" (Life Technologies GmbH) transformiert, in diesen Zellen vermehrt und die DNA-Sequenz der isolierten Plasmide mittels Sequenzierung verifiziert. Zwei der insgesamt 4 möglichen Konstrukte, welche auf diese Art und Weise generiert wurden, tragen die Bezeichnungen pAF-ts-pyrH und pAF-ts-plsC (siehe Fig. 2 und 3) .

### Beispiel 3: Transformation ausgewählter E. coli Stämme mit pAF-ts-pyrH oder pAF-ts-plsC

Die in Beispiel 2 beschriebenen Plasmide pAF-ts-pyrH und pAF-ts-plsC mit temperatur-sensitivem Replikationsursprung wurden mit der dem Fachmann bekannten CaCl₂-Methode in die beiden E. *coli* Stämme W3110 (ATCC 27325) und W3110lpp3 (beschrieben in US2008076158 A1 als "leaky"-Stamm) transformiert. Die Selektion der transformierten Zellen erfolgte auf LB-Agarplatten, die 100 mg/l Ampicillin enthielten. Die auf diese Art und Weise generierten Stämme tragen die Bezeichnungen W3110/pAF-ts-pyrH, W3110lpp3/pAF-ts-pyrH, W3110/pAF-ts-plsC und W3110lpp3/pAF-ts-plsC.

### Beispiel 4: Deletion des Gens pyrH (Inaktivierung Uridylatkinase) bzw. des Gens plSC (Inaktivierung 1-Acylglycerol-3-Phosphat O-Acyltransferase) in E. coli

### A) Deletion des Gens pyrH

Das Gen *pyrH,* welches in *E. coli* für das Enzym Uridylatkinase (PyrH) kodiert, wurde nach der von Datsenko und Wanner entwickelten "λ-Red-Methode" in den *E*. *coli* Stämmen W3110/pAF-ts-pyrH und W3110lpp3/pAF-ts-pyrH deletiert (Datsenko und Wanner, 2000, P.N.A.S. 97: 6640-6645). Ein DNA-Fragment, welches für das Kanamycin-Resistenzmarkergen (*kanR*) codiert, wurde mit den Primern pyrH-fw (SEQ ID No. 9) und pyrH-rev (SEQ ID No. 10) amplifiziert. Der Primer pyrH-fw kodiert für eine Sequenz, bestehend aus 30 Nukleotiden, welche homolog zum 5'-Ende des *pyrH*-Gens ist, und eine 20 Nukleotide umfassende Sequenz, die komplementär zu einer DNA-Sequenz ist, welche eine der beiden FRT-sites (FLP-recognition target) auf dem Plasmid pKD13 (Coli Genetic Stock Center (CGSC) No. 7633) kodiert. Der Primer pyrH-rev kodiert für eine Sequenz, bestehend aus 30 Nukleotiden, welche homolog zum 3'-Ende des *p*yrH-Gens ist, und eine 20 Nukleotide umfassende Sequenz, die komplementär zu einer DNA-Sequenz ist, welche die zweite FRT-site auf dem Plasmid pKD13 kodiert.

Das amplifizierte PCR-Produkt wurde mittels Elektroporation in die *E. coli* Stämme W3110/pAFts-pyrH und W3110lpp3/pAF-ts-pyrH (siehe Beispiel 3) eingebracht. Die Selektion auf Zellen mit chromosomaler Integration des Kanamycin-Resistenz-Markergens (*kanR*) erfolgte auf LB-Agarplatten, die 50 mg/l Kanamycin und 100 mg/l Ampicillin enthielten. Die Entfernung des chromosomal eingebrachten Kanamycin-Resistenz-Markergens (*kanR*) erfolgte mit dem Enzym FLP-Rekombinase, welches auf dem Plasmid pCP20 (CGSC No. 7629) kodiert ist. Die Selektion auf pCP20-enthaltende Zellen erfolgte auf LB-Agarplatten, die 100 mg/l Ampicillin (Selektion auf pAF-ts-pyrH) und 34 mg/l Chloramphenicol (Selektion auf pCP20) enthielten. Aufgrund eines temperatursensitiven Replikationsursprunges (ori) kann das Plasmid pCP20 nach erfolgter Transformation durch Kultivierung der *E. coli* Zellen bei nicht permissiver, d.h. erhöhter Temperatur, z.B. bei 42°C, wieder entfernt werden.

Eine erste Selektion auf Verlust des temperatursensitiven Plasmids pCP20 bei gleichzeitigem Erhalt des temperatur-sensitiven Plasmids pAF-ts-pyrH, erfolgte auf LB-Agarplatten, die 100 mg/l Ampicillin enthielten (Selektion auf pAF-ts-pyrH). Im weiteren Verlauf wurden die vorselektierten Ampicillin-resistenten Bakterien-Klone auf Kanamycin-Sensitivität, d.h. den Verlust des chromosomal eingebrachten Kanamycin-Markergens, sowie auf Chloramphenicol-Sensitivität, d.h. den Verlust des temperatursensitiven Plasmids pCP20 überprüft.

Nur Kanamycin-, sowie Chloramphenicol-sensitive aber Ampicillin-resistente Klone wurden abschließend mit den Primern pyrH-check-for (SEQ ID No. 11) und pyrH-check-rev (SEQ ID No. 12) auf die chromosomale Deletion des *pyrH*-Gens hin überprüft. Als Template für die Überprüfung der chromosomalen pyrH-Deletion mittels PCR diente chromosomale DNA der selektierten Ampicillin-resistenten, Chloramphenicol- und Kanamycin-sensitiven Klone.

Die auf diese Art und Weise generierten und überprüften Ampicillin-resistenten *E. coli*-Stämme mit chromosomaler *pyrH*-Deletion und Plasmid-kodierter *pyrH*-Expression tragen die Bezeichnungen W3110ΔpyrH/pAF-ts-pyrH und W3110lpp3ΔpyrH/pAF-ts-pyrH.

### B) Deletion des plsC-Gens

Analog zum *pyrH*-Gen wurde das Gen *plsC,* welches in *E. coli* für das Enzym 1-Acylglycerol-3-Phosphat O-Acyltransferase (PlsC) kodiert, in den E. *coli* Stämmen W3110/pAF-ts-plsC und W3110lpp3/pAF-ts-plsC deletiert (Datsenko und Wanner, 2000, P.N.A.S. 97: 6640-6645). Ein DNA-Fragment, welches für das Kanamycin-Resistenzmarkergen (*kanR*) kodiert, wurde mit den Primern plsC-fw (SEQ ID No. 13) und plsC-rev (SEQ ID No. 14) amplifiziert. Der Primer plsC-fw kodiert für eine Sequenz, bestehend aus 30 Nukleotiden, welche homolog zum 5'-Ende des *plsC*-Gens ist, und eine 20 Nukleotide umfassende Sequenz, die komplementär zu einer DNA-Sequenz ist, welche eine der beiden FRT-sites (FLP-recognition target) auf dem Plasmid pKD13 (Coli Genetic Stock Center (CGSC) No. 7633) kodiert. Der Primer plsC-rev kodiert für eine Sequenz, bestehend aus 30 Nukleotiden, welche homolog zum 3'-Ende des *plsC*-Gens ist, und eine 20 Nukleotide umfassende Sequenz, die komplementär zu einer DNA-Sequenz ist, welche die zweite FRT-site auf dem Plasmid pKD13 kodiert.

Das amplifizierte PCR-Produkt wurde mittels Elektroporation in die *E*. *coli* Stämme W3110/pAFts-plsC und W3110lpp3/pAF-ts-plsC (siehe Beispiel 3) eingebracht. Die Entfernung des chromosomal eingebrachten Kanamycin-Resistenz-Markergens (*kanR*) erfolgte wieder mit dem Enzym FLP-Rekombinase (kodiert auf Plasmid pCP20). Die Selektion auf pCP20-enthaltene Zellen erfolgte auch hier auf LB-Agarplatten, die 100 mg/l Ampicillin (Selektion auf pAF-ts-plsC) und 34 mg/l Chloramphenicol (Selektion auf pCP20) enthielten. Eine erste Selektion auf Verlust des temperatursensitiven Plasmids pCP20 bei gleichzeitigem Erhalt des temperatur-sensitiven Plasmids pAF-ts-plsC, erfolgte auf LB-Agarplatten, die 100 mg/l Ampicillin enthielten (Selektion auf pAF-ts-plsC). Im weiteren Verlauf wurden die vorselektierten Ampicillin-resistenten Bakterien-Klone auf Kanamycin-Sensitivität, d.h. den Verlust des chromosomal eingebrachten Kanamycin-Markergens, sowie auf Chloramphenicol-Sensitivität, d.h. den Verlust des temperatursensitiven Plasmids pCP20 überprüft. Diese Klone wurden abschließend mit den Primern plsC-check-for (SEQ ID No. 15) und plsC-check-rev (SEQ ID No. 16) auf die chromosomale Deletion des *plsC*-Gens hin überprüft. Als Template für die Überprüfung der chromosomalen *plsC*-Deletion mittels PCR diente chromosomale DNA der selektierten Ampicillin-resistenten, Chloramphenicol- und Kanamycin-sensitiven Klone.

Die auf diese Art und Weise generierten und überprüften Ampicillin-resistenten *E. coli*-Stämme mit chromosomaler *plsC*-Deletion und Plasmid-kodierter *plsC*-Expression tragen die Bezeichnungen W3110ΔplsC/pAF-ts-plsC und W3110lpp3ΔplsC/pAF-ts-plsC.

### Beispiel 5: Generierung eines Produktionsplasmides mit Antibiotikaresistenzgen für die Produktion von Cystein

Als Ausgangsplasmide für die Klonierung und Expression der Gene *cysEX* (kodiert für feedbackresistente Variante der Serin Acyltransferase; CysE) und *orf306* (kodiert für O-Acetylserin / Cystein-Exporter; EamA) dienten das Basisplasmid pMT1 sowie das in EP0885962B1 beschriebene Produktionsplasmid pACYC184-LH-cysEX-orf306.

pMT1 enthält neben dem Tetracyclin-Resistenzgen (*tetR*) noch den tac-Promotor, der durch das LacIq-Genprodukt, dessen Gen ebenfalls auf dem Plasmid vorliegt, reprimiert wird und der durch einen Induktor wie z.B. D-Lactose oder Isopropyl-β-D-thiogalactopyranosid (IPTG) angeschaltet werden kann.

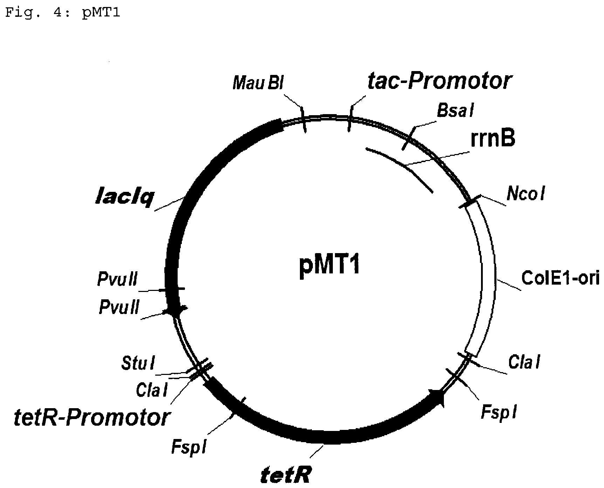

Eine Restriktions- und Funktionskarte von Plasmid pMT1 ist in Fig. 4 gezeigt. Die Sequenz des Plasmides pMT1 ist im Sequenzprotokoll (SEQ ID No. 17) hinterlegt.

Für die Generierung eines neuen Produktionsplasmides für die Produktion von Cystein, auf Basis von pMT1, wurde ein NcoI-BsaBI-Fragment des Plasmides pACYC184-LH-cysEX-orf306 (beschrieben in EP0885962 B1), welches für die Gene *cysEX* und *orf306* kodiert, mit einem 2458 bp großen NcoI-PvuII-Fragment (kodiert für ColE1-ori und Tetracyclinresistenz, tetR) des Plasmides pMT1 ligiert.

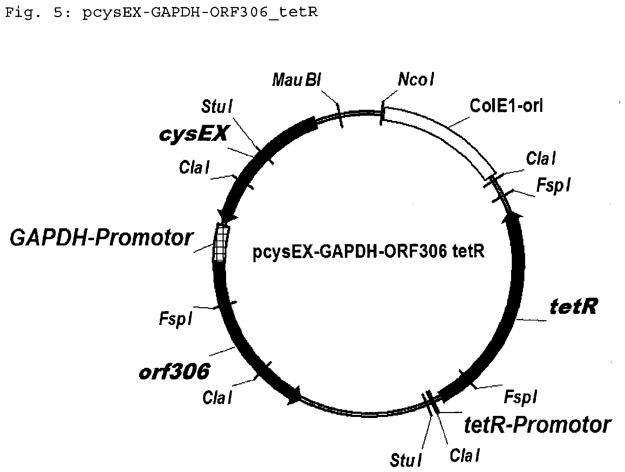

Der Ligationsansatz wurde in "DH5α™-T1R *E. coli*-Zellen" (Life Technologies GmbH) transformiert, in diesen Zellen vermehrt und die DNA-Sequenz der isolierten Plasmide mittels Sequenzierung verifiziert. Das resultierende Expressionsplasmid trägt die Bezeichnung pcysEX-GAPDH-ORF306_tetR (siehe Fig. 5).

### Beispiel 6: Generierung eines Produktionsplasmides mit Antibiotikaresistenzgen für die Produktion von α-CGTase

Als Ausgangsplasmide für die Klonierung und Expression des Cyclodextrin-Glycosyl-Transferase (CGTase)-Gens aus *Klebsiella pneumoniae* M5a1 (Genbanknr. M15264) dienten erneut das Plasmid pMT1 sowie das in US2008076158 A1 beschriebene Plasmid pCGT. Für die Generierung eines neuen Produktionsplasmides für die Produktion der CGTase, auf Basis von pMT1, wurde ein MauBI-BsaI-Fragment des Plasmides pCGT, welches für das CGTase-Gen aus *Klebsiella pneumoniae* M5a1 kodiert, mit einem 4004 bp großen MauBI-BsaI-Fragment des Plasmides pMT1 ligiert. Dieses 4004 bp große Fragment des Plasmides pMT1 kodiert für den ColE1-ori, den lac/tac-Operator und das Tetracylinresistenzgen (*tetR*).

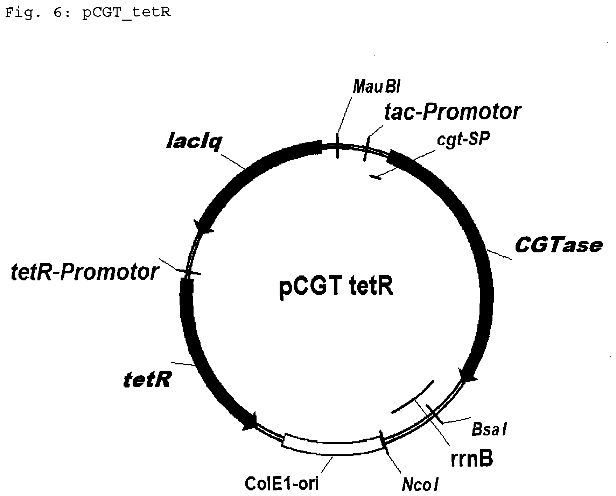

Der Ligationsansatz wurde in "DH5α™-T1R *E. coli*-Zellen" (Life Technologies GmbH) transformiert, in diesen Zellen vermehrt und die DNA-Sequenz der isolierten Plasmide mittels Sequenzierung verifiziert. Das resultierende Expressionsplasmid trägt die Bezeichnung pCGT_tetR (siehe Fig. 6).

### Beispiel 7: Generierung eines Produktionsplasmids mit Antibiotikaresistenzgen für die Produktion von Fab-anti-Lysozym

Als Ausgangsplasmide für die Klonierung und Expression der Gene des Anti-Lysozym-Fab-Fragments dienten erneut das Plasmid pMT1 sowie das in US20080076158 A1 beschrieben pFab-anti-Lysozym. Für die Generierung eines neuen Produktionsplasmids für die Produktion des Antikörperfragmentes Fab-anti-Lysozym, auf Basis von pMT1, wurde ein MauBI-BsaI-Fragment des Plasmids Fab-anti-Lysozym, welches für die beiden Ketten, d.h. die schwere Kette (V_{H}-C_{H}1-Domänen) und die leichte Kette (V_{L}-C_{L}-Domänen) des Anti-Lysozym-Fab-Fragments kodiert, mit einem 4004 bp großen MauBI-BsaI-Fragment des Plasmides pMT1 ligiert. Dieses 4004 bp große Fragment des Plasmides pMT1 kodiert für den ColE1-ori, den lac/tac-Operator und das Tetracylinresistenzgen (*tetR*).

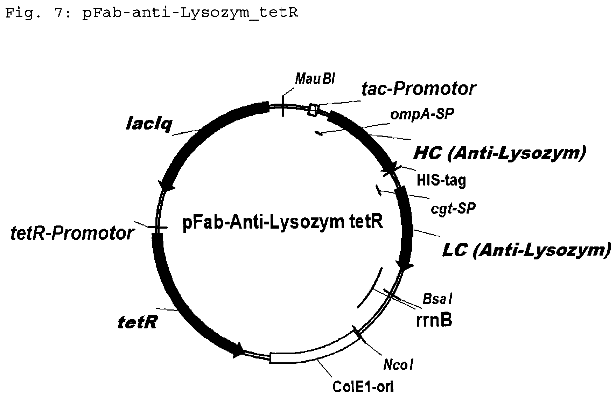

Der Ligationsansatz wurde in "DH5α™-T1R *E. coli*-Zellen" (Life Technologies GmbH) transformiert, in diesen Zellen vermehrt und die DNA-Sequenz der isolierten Plasmide mittels Sequenzierung verifiziert. Das resultierende Expressionsplasmid trägt die Bezeichnung pFab-anti-Lysozym_tetR (siehe Fig. 7).

### Beispiel 8: Generierung von Produktionsplasmiden mit den Markergenen pyrH oder plsC als Grundlage für die Erstellung erfindungsgemäßer Produktionsplasmide ohne Antibiotikaresistenzgen

Als Ausgangsplasmide für die Generierung von Produktionsplasmiden mit *pyrH* oder *plsC* als Markergen dienten die in den Beispielen 5 bis 7 beschriebenen Plasmide pcysEX-GAPDH-ORF306_tetR, pCGT_tetR und pFab-anti-Lysozym_tetR. All diese Plasmide besitzen eine einzelne NcoI-Restriktionsschnittstelle (siehe Abbildungen 5 bis 7). Diese universelle NcoI-Schnittstelle wurde für die Klonierung bzw. Integration der Gene *pyrH* oder *plsC* genutzt.

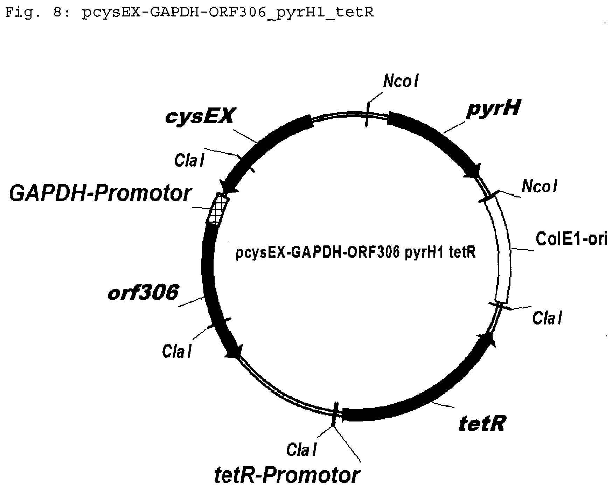

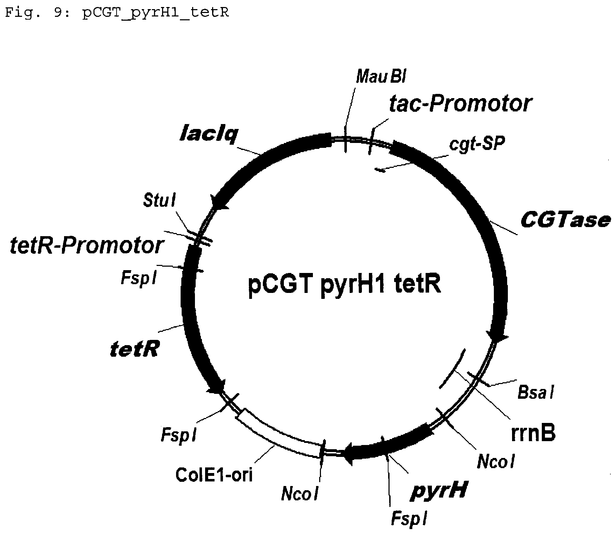

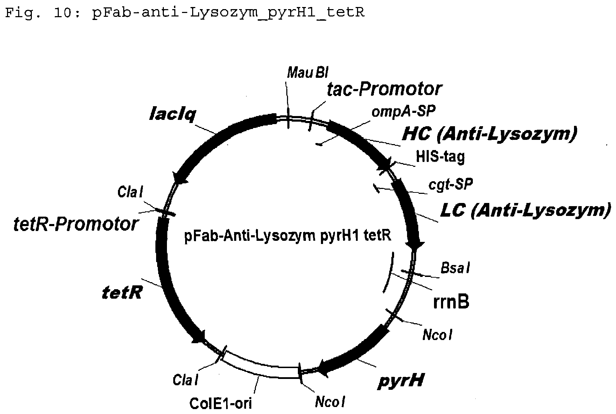

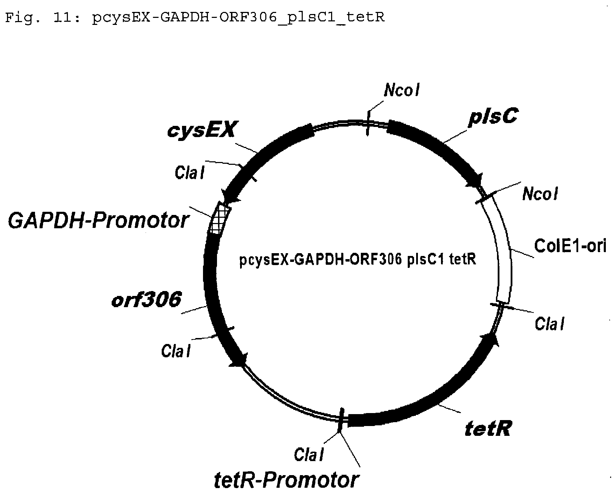

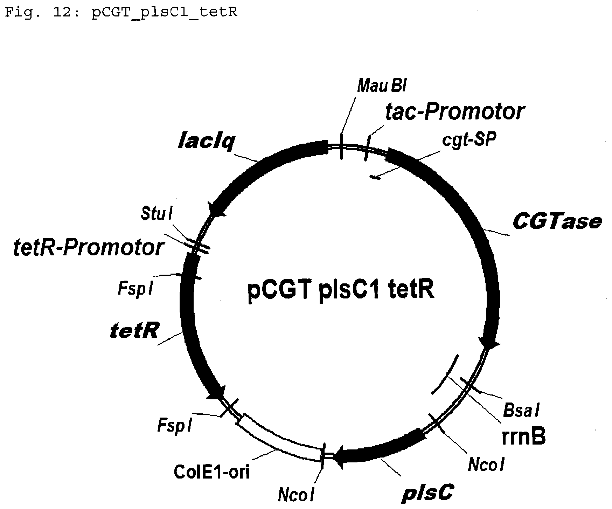

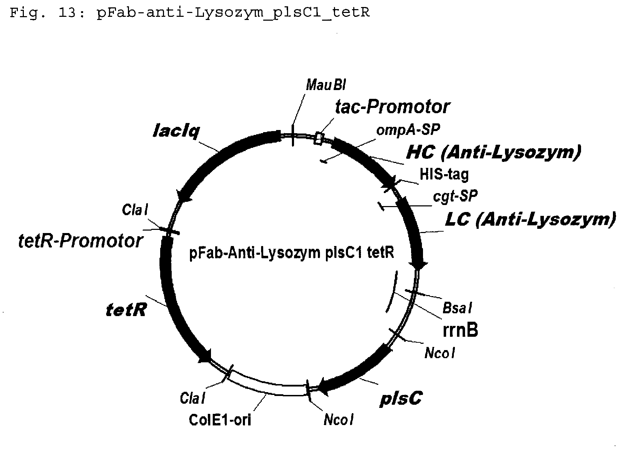

Hierfür wurden die in Beispiel 1 beschriebenen, und mit dem Restriktionsenzym NcoI geschnittenen PCR-Produkte mit den Plasmiden pcysEX-GAPDH-ORF306_tetR, pCGT_tetR und pFab-anti-Lysozyme_tetR ligiert, nachdem diese ebenfalls mit NcoI geschnitten wurden. Die einzelnen Ligationsansätze wurden in "DH5α™-T1R *E. coli*-Zellen" (Life Technologies GmbH) transformiert, in diesen Zellen vermehrt und die DNA-Sequenz der isolierten Plasmide mittels Sequenzierung verifiziert. Die resultierenden Konstrukte tragen, je nach Orientierung und verwendeten Markergen, folgende Bezeichnungen:
- pcysEX-GAPDH-ORF306_pyrH1_tetR (siehe Fig. 8) und pcysEX-GAPDH-ORF306_pyrH2_tetR
- pCGT_pyrH1_tetR (siehe Fig. 9)und pCGT_pyrH2_tetR
- pFab-anti-Lysozyme_pyrH1_tetR (siehe Fig. 10) und pFab-anti-Lysozyme_pyrH2_tetR
- pcysEX-GAPDH-ORF306_plsC1_tetR (siehe Fig. 11) und pcysEX-GAPDH-ORF306_plsC2_tetR
- pCGT_plsC1_tetR (siehe Fig. 12) und pCGT_plsC2_tetR
- pFab-anti-Lysozyme_plsC1_tetR (siehe Fig. 13) und pFab-anti-Lysozyme_plsC2_tetR

Für die finale Entfernung des tetR-Gens wurde mit den Varianten pcysEX-GAPDH-ORF306_pyrH1_tetR, pCGT_pyrH1_tetR, pFab-anti-Lysozyme_pyrH1_tetR, pcysEX-GAPDH-ORF306_plsC1_tetR, pCGT_plsC1_tetR und pFab-anti-Lysozyme_plsC1_tetR weitergearbeitet (siehe Fig. 8 bis 13).

### Beispiel 9: Entfernung des Antibiotka-Resistenzgens tetR und Transformation der Produktionsplasmide mit pyrH bzw. plsC als verbleibendem Markergen in E. coli Stämme mit chromosomaler pyrH- bzw. plsC-Deletion

Als Ausgangsplasmide für die Generierung von Produktionsplasmiden ohne das Antibiotikaresistenzgen tetR und mit *pyrH* oder *plsC* als Markergen dienten die in Beispiel 8 beschriebenen Plasmide pcysEX-GAPDH-ORF306_pyrH1_tetR, pCGT_pyrH1_tetR, pFab-anti-Lysozyme_pyrH1_tetR, pcysEX-GAPDH-ORF306_ plsC1_tetR, pCGT_plsC1_tetR und pFab-anti-Lysozyme_ plsC1_tetR. Die Entfernung des Antibiotikaresistenzgens tetR von den in Beispiel 8 beschriebenen Plasmiden pFab-anti-Lysozyme_ pyrH1_tetR und pFab-anti-Lysozym_plsc1_tetR erfolgte über einen Verdau mit dem Restriktionsenzym ClaI und anschließender Religation.

Für die Plasmide pcysEX-GAPDH-ORF306_pyrH1_tetR bzw. pcysEX-GAPDH-ORF306_plsC1_tetR wurde ähnlich verfahren, nur dass das Gen tetR über einen Partialverdau mit ClaI entfernt wurde, da sich zwei weitere ClaI-Schnittstellen in den Strukturgenen *cysEX* und *orf306* befinden (siehe Fig. 8 und 11).

Im Falle von pCGT_plsC1_tetR wurde das *tetR*-Gen über einen Verdau mit den Enzymen StuI (schneidet blunt End) und FspI (schneidet blunt End) vom Plasmid entfernt.

Im Falle von pCGT_pyrH1_tetR wurde das tetR-Gen ebenfalls über einen Partialverdau mit den Enzymen StuI (schneidet blunt End) und FspI (schneidet blunt End) entfernt, da sich eine weitere FspI-Schnittstelle im *pyrH*-Gen befindet (siehe Fig. 9).

Die jeweiligen linearen Vektorfragmente ohne tetR wurden nach dem Restriktionsverdau über eine Agarose-Gelelektrophorese gereinigt und mit dem "QIAquick Gel Extraction Kit" (Qiagen GmbH, Hilden, D) nach Angaben des Herstellers aus dem Agarosegel isoliert. Anschließend wurde das jeweilige tetRfreie Vektorfragment religiert.

Die entsprechenden Ligationsansätze wurden mit einer abgewandelten CaCl₂-Methode in die im Beispiel 4 beschriebenen Stämme W3110ΔpyrH/pAF-ts-pyrH bzw. W3110lpp3ΔpyrH/pAF-ts-pyrH oder W3110ΔpyrH/pAF-ts-plsC bzw. W3110lpp3ΔpyrH/pAF-ts-plsC transformiert. Für die Transformation, d.h. die Einbringung der Antibiotikaresistenz-freien Produktionsplasmide mit *pyrH* oder *plsC* als Markergen in E. *coli* Stämme mit entsprechender chromosomaler Deletion (*pyrH* oder *plsC*) wurde folgendermaßen verfahren:
Nach der Zugabe von 5 bis 20 µl des jeweiligen Ligationsansatzes zu 100 µl CaCl₂-kompetenter Zellen der Stämme W3110ΔpyrH/pAF-ts-pyrH und W3110lpp3ΔpyrH/pAF-ts-pyrH bzw. W3110ΔplsC/pAF-ts-plsC und W3110lpp3ΔplsC/pAF-ts-plsC wurden die Zellen für weitere 30 Minuten auf Eis inkubiert. Nach einem kurzzeitigen Hitzeschock für 45 Sekunden bei 42°C wurden die Zellen 2 min auf Eis abgekühlt. Anschließend wurden dem Transformationsansatz 900 µl LB-Medium zugesetzt und die Zellen, nicht wie üblich bei 37°C, sondern 30 bis 90 min bei 47°C bis 55°C inkubiert / regeneriert. Die weitere Inkubation bei erhöhter nicht permissiver Temperatur erfolgte dann für 15 bis 24 h bei 40-45°C auf LB-Agarplatten oder in flüssigem LB-Medium ohne Antibiotikum (z.B. ohne Tetracyclin).

Die 30 - 90 minütige Regenerationsphase bei 47°C bis 55°C sowie die Kultivierung für 15 bis 24 h bei erhöhter, nicht permissiver Temperatur erleichtert den Austausch der temperatursensitiven Plasmide pAF-ts-pyrH bzw. pAF-ts-plsC gegen das finale, Antibiotikaresistenz-freie Produktionsplasmid mit *pyrH* oder *plsC* als neuem Selektionsmarkergen.

Die besten Transformations-Ergebnisse wurden erzielt, wenn die transformierten Zellen 60 min bei 52°C regeneriert und anschließend 20 h bei 42°C auf LB-Agarplatten inkubiert wurden. Eine Vorab-Selektion auf Verlust des temperatursensitiven Plasmids pAF-ts-pyrH bzw. pAF-ts-plsC bei gleichzeitigem Austausch gegen das jeweilige *pyrH-* oder *plsC*-haltige Produktionsplasmid ohne Tetracyclinresistenzgen tetR erfolgte zunächst auf LB-Agarplatten ohne Antibiotikum.

Anschließend wurden die vorselektierten Bakterien-Klone auf Ampicillin-Sensitivität, d.h. den Verlust des temperatursensitiven Plasmides pAF-ts-pyrH bzw. pAF-ts-plsC überprüft.

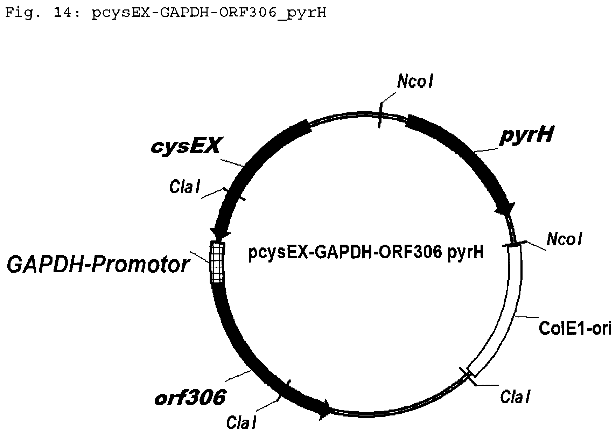

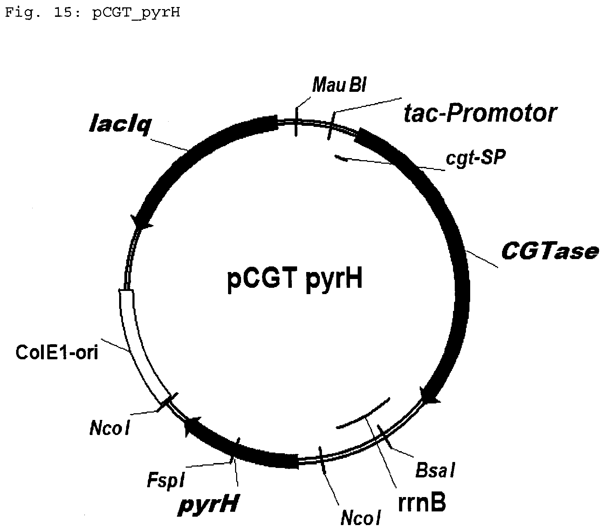

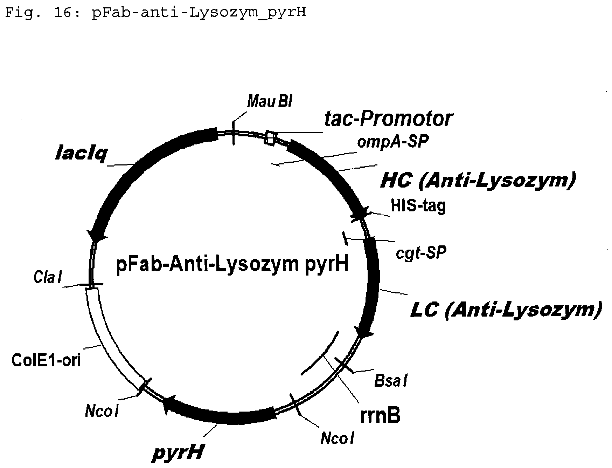

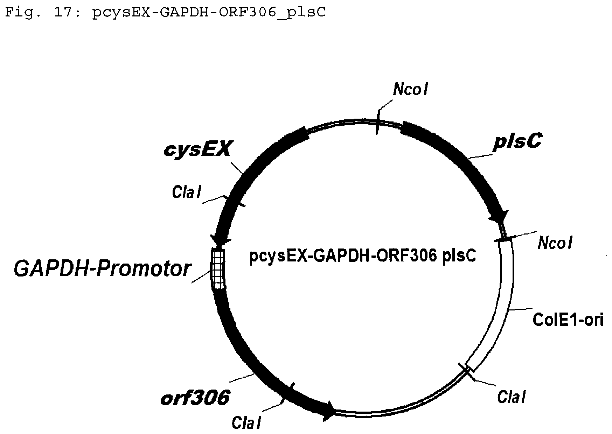

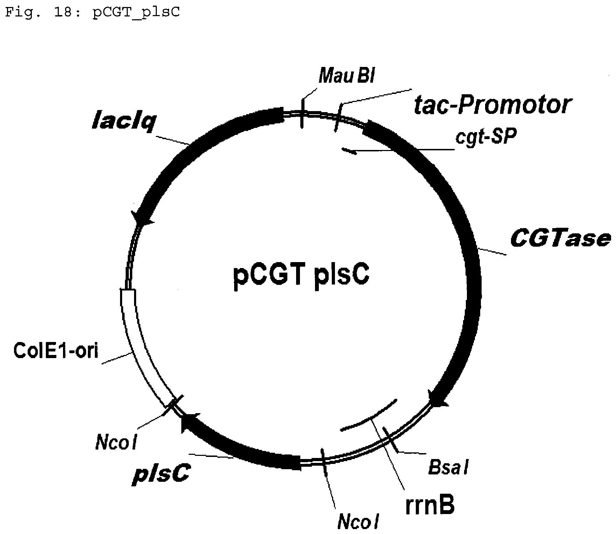

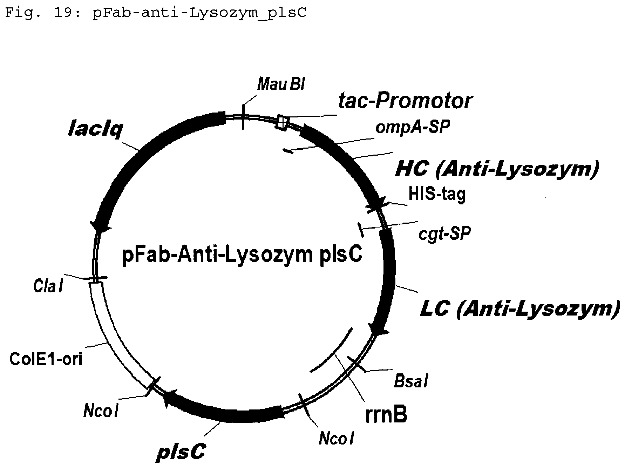

Die *pyrH-* oder *plsC*-kodierenden Produktionsplasmide aus Ampicillin-sensitiven Klonen wurden abschließend mittels Restriktionsverdau überprüft. Restriktionskarten der *pyrH*-kodierenden Produktionsplasmide pcysEX-GAPDH-ORF306_pyrH, pCGT_pyrH und pFab-anti-Lysozyme_pyrH, jeweils ohne das Antibiotikaresistenzgen tetR, sind in den Abbildungen 14 bis 16 dargestellt. Restriktionskarten der *plsC*-kodierenden Produktionsplasmide pcysEX-GAPDH-ORF306_plsC, pCGT_plsC und pFab-anti-Lysozyme_plsC, jeweils ohne das Antibiotikaresistenzgen *tetR*, sind in den Abbildungen 17 bis 19 dargestellt.

Die auf diese Art und Weise generierten und überprüften Antibiotikaresistenz-freien *E. coli*-Stämme mit *pyrH-* oder *plsC*-kodierendem Produktionsplasmid und chromosomaler *pyrH-* bzw. *plsC*-Deletion tragen die Bezeichnungen:
- W3110ΔpyrH/pcysEX-GAPDH-ORF306_pyrH
- W3110lpp3ΔpyrH/pCGT_pyrH
- W3110lpp3ΔpyrH/pFab-anti-Lysozym_pyrH
- W3110ΔplsC/pcysEX-GAPDH-ORF306_plsC
- W3110lpp3ΔplsC/pCGT_plsC
- W3110lpp3ΔplsC/pFab-anti-Lysozym_plsC

### Beispiel 10: Cystein-Fermentation

### Vorkultur 1:

20 ml LB-Medium wurden in einem Erlenmeyerkolben (100 ml) mit dem jeweiligen *E. coli* Stamm W3110/pcysEX-GAPDH-ORF306_tetR, W3110ΔpyrH/pcysEX-GAPDH-ORF306_pyrH oder W3110ΔplsC/pcysEX-GAPDH-ORF306_plsC beimpft und für sieben Stunden auf einem Schüttler (150 rpm, 30°C) inkubiert. Für eine Kultivierung von Konstrukt W3110/pcysEX-GAPDH-ORF306_tetR im Sinne des Stands der Technik, d.h. mit Antibiotikum als Selektionsmittel, wurde das Medium mit 15 mg/L Tetracyclin supplementiert.

### Vorkultur 2:

Anschließend wurde die Vorkultur 1 vollständig in 100 ml SM1-Medium überführt (12 g/l K₂HPO₄, 3 g/l KH₂PO₄, 5 g/l (NH₄)₂SO₄, 0,3 g/l MgSO₄ x 7 H₂O, 0,015 g/l CaCl₂ x 2 H₂O, 0,002 g/l FeSO₄ x 7 H₂O, 1 g/l Na3Citrat x 2 H₂O, 0,1 g/l NaCl, 1 ml/l Spurenelementlösung, bestehend aus 0,15 g/l Na₂MoO₄ x 2H₂O, 2,5 g/l H₃BO₃, 0,7 g/l CoCl₂ x 6 H₂O, 0,25 g/l CuSO₄ x 5 H₂O, 1,6 g/l MnCl₂ x 4 H₂O, 0,3 g/l ZnSO₄ x 7 H₂O), das mit 5 g/l Glucose und 5 mg/l Vitamin B1 supplementiert war. Die Kulturen wurden in Erlenmeyerkolben (1 l) bei 30 °C für 17 h mit 150 rpm geschüttelt. Nach dieser Inkubation lag die optische Dichte bei 600 nm (OD₆₀₀) zwischen 3 und 5. Für die Kultivierung von W3110/pcysEX-GAPDH-ORF306_tetR im Sinne des Stands der Technik, d.h. mit Antibiotikum als Selektionsmittel, wurde das Medium mit 15 mg/L Tetracyclin supplementiert.

### Hauptkultur:

Die Fermentation wurde in Fermentern des Typs BIOSTAT B der Firma Sartorius Stedim durchgeführt. Es wurde ein Kulturgefäß mit 2 l Gesamtvolumen verwendet. Das Fermentationsmedium (900 ml) enthält 15 g/l Glucose, 10 g/l Trypton (Difco), 5 g/l Hefeextrakt (Difco) , 5 g/l (NH₄)₂SO₄, 1,5 g/l KH₂PO₄, 0,5 g/l NaCl, 0,3 g/l MgSO₄ x 7 H₂O, 0,015 g/l CaCl₂ x 2 H₂O, 0,075 g/l FeSO₄ x 7 H₂O, 1 g/l Na₃ Citrat x 2 H₂O und 1 ml Spurenelementlösung (siehe oben) und 0,005 g/l Vitamin B1. Der pH-Wert im Fermenter wurde zu Beginn durch Zupumpen einer 25% NH₄OH-Lösung auf 6,5 eingestellt. Während der Fermentation wurde der pH-Wert durch automatische Korrektur mit 25% NH₄OH auf einem Wert von 6,5 gehalten. Zum Animpfen wurden 100 ml der Vorkultur 2 in das Fermentergefäß gepumpt. Das Anfangsvolumen betrug somit etwa 1 l.

Die Kulturen wurden zu Beginn mit 400 rpm gerührt und mit 2 vvm einer über einen Sterilfilter entkeimten Druckluft begast. Unter diesen Startbedingungen war die Sauerstoff-Sonde vor der Inokulation auf 100% Sättigung kalibriert worden. Der Soll-Wert für die O₂-Sättigung während der Fermentation wurde auf 50 % eingestellt. Nach Absinken der O₂-Sättigung unter den Soll-Wert wurde eine Regulationskaskade gestartet, um die O₂-Sättigung wieder an den Soll-Wert heranzuführen. Dabei wurde zunächst die Gaszufuhr kontinuierlich erhöht (auf max. 5 vvm) und anschließend die Rührgeschwindigkeit (auf max. 1.500 rpm) kontinuierlich gesteigert.

Die Fermentation wurde bei einer Temperatur von 30 °C durchgeführt. Nach 2 h Fermentationszeit erfolgte die Zufütterung einer Schwefelquelle in Form einer sterilen 60 % Natrium-Thiosulfat x 5 H₂O - Stammlösung mit einer Rate von 1,5 ml pro Stunde. Sobald der Glukose-Gehalt im Fermenter von anfänglich 15 g/l auf ca. 2 g/l abgesunken war, erfolgte eine kontinuierliche Zudosierung einer 56% Glukose-Lösung. Die Fütterungsrate wurde so eingestellt, dass die Glukosekonzentration im Fermenter 2 g/l fortan nicht mehr überstieg.

Die Glukose-Bestimmung wurde mit einem Glukoseanalysator der Firma YSI (Yellow Springs, Ohio, USA) durchgeführt. Für die Fermentation von Konstrukt W3110/pcysEX-GAPDH-ORF306_tetR im Sinne des Stands der Technik, d.h. mit Antibiotikum als Selektionsmittel, wurde das Medium mit 15 mg/L Tetracyclin supplementiert.

Die Fermentationsdauer betrug 48 Stunden. Danach wurden Proben entnommen und der Gehalt von L-Cystein und den davon abgeleiteten Derivaten im Kulturüberstand (v.a. L-Cystein und Thiazolidin) und im Niederschlag (L-Cystin) jeweils getrennt voneinander bestimmt. Zu diesem Zweck wurde jeweils der colorimetrische Test von Gaitonde verwendet (Gaitonde, M. K. (1967), Biochem. J. 104, 627-633). Das im Niederschlag befindliche L-Cystin musste zuerst in 8% Salzsäure aufgelöst werden, bevor es auf dieselbe Art quantifiziert werden konnte. Die in Tabelle 1 aufgeführten Werte für Gesamtcystein entsprechen der Summe aus den L-Cystein im Kulturüberstand und L-Cystin im Niederschlag. Dabei entspricht jedes Molekül L-Cystin zwei Molekülen L-Cystein.

**Tabelle 1: Gehalt an Gesamtcystein (L-Cystein_{Kulturüberstand} + L-Cystin_{Niederschlag}) in der Kulturbrühe nach 48 h und Stabilität der Produktionsplasmide**

| ±±Stamm | Gesamtcystein (g/L) | Plasmidstabilität |
|---|---|---|
| W3110/pcysEX-GAPDH-ORF306_tet^{R} (kultiviert mit Tetracyclin) * | 19,0 ± 0,4 | 95% ± 5% |
| W3110/pcysEX-GAPDH-ORF306_tet^{R} (kultiviert ohne Tetracyclin) | 10,0 ± 4,1 | 60% ± 19% |
| W3110ΔpyrH x pcysEX-GAPDH-ORF306_pyrH1** | 20,4 ± 0,3 | 97% ± 3% |
| W3110ΔplsC/pcysEX-GAPDH-ORF306 plsC** | 21,1 ± 0,3 | 95% ± 5% |

| | | |
|---|---|---|
| **^{*}Konstrukt im Sinne des Stands der Technik (Vergleichsbeispiel)** ****erfindungsgemäßes Konstrukt** | | |

### Beispiel 11: Sekretorische Produktion einer Cyclodextrin-Glycosyl-Transferase im 10 1-Maßstab (Fermentation)

Mit Hilfe einer *lpp*-Mutante von *E*. *coli* können biotechnologisch relevante Enzyme wie beispielsweise CGTasen produziert und ins Medium sekretiert werden (US2008076158 A1).

Die sekretorische Produktion der CGTase erfolgte in 10 L Rührkesselfermentern mit den Stämmen W31101pp3/pCGT_tetR (Kontrolle), W3110lpp3ΔpyrH/pCGT_pyrH und W3110lpp3ΔpyrH/pCGT_plsC. Der mit 6 l des Fermentationsmediums FM4 (1,5 g/l KH₂PO₄; 5 g/l (NH₄)₂SO₄; 0,5 g/l MgSO₄ x 7 H₂O; 0,15 g/l CaCl₂ x 2 H₂O, 0,075 g/l FeSO₄ x 7 H₂O; 1 g/l Na₃ Citrat x 2 H₂O; 0,5 g/l NaCl; 1 ml/l Spurenelementlösung (0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O); 5 mg/l Vitamin B₁; 3 g/l Phyton; 1,5 g/l Hefeextrakt; 10 g/l Glukose) befüllte Fermenter wurde im Verhältnis 1:10 mit einer Vorkultur, die über Nacht im gleichen Medium kultiviert wurde, angeimpft. Während der Fermentation wurde eine Temperatur von 30 °C eingestellt und der pH-Wert durch Zudosierung von NH₄OH bzw. H₃PO₄ bei einem Wert von 7,0 konstant gehalten. Glukose wurde über die Fermentation hinweg zudosiert, wobei eine maximale Glukose-Konzentration im Fermentationsmedium von < 10 g/l angestrebt wurde. Die Induktion der Expression erfolgte durch Zugabe von Isopropyl-β-D-thiogalactopyranosid (IPTG) ad 0,1 mM am Ende der logarithmischen Wachstumsphase.

Für die Fermentation des Konstruktes W3110lpp3/pCGT_tetR im Sinne des Stands der Technik, d.h. mit Antibiotikum als Selektionsmittel, wurde das Medium mit 15 mg/L Tetracyclin supplementiert.

Nach 72 h Fermentation wurden Proben entnommen, die Zellen durch Zentrifugation vom Fermentationsmedium abgetrennt und der CGTase-Gehalt im Fermentationsüberstand, wie in Beispiel 4 von US2008076158A1 beschrieben, bestimmt.

In Tabelle 2 sind die Ausbeuten an funktioneller CGTase sowie die Aktivitäten im Fermentationsüberstand aufgelistet.

**Tabelle 2: Cyclodextrin-Glycosyltransferase-Ausbeuten im Fermentationsüberstand nach 72 Stunden Fermentation**

| Stamm | CGTase (U/ml) | CGTase (mg/l) | Plasmidstabilität |
|---|---|---|---|
| W31101pp3/pCGT_tetR (kulti-viert mit Tetracyclin)* | 555 ± 15 | 3750 ± 145 | 97% ± 3% |
| W3110lpp3/pCGT_tetR (kulti-viert ohne Tetracyclin) | 340 ± 65 | 2210 ± 425 | 55% ± 21% |
| W3110lpp3ΔpyrH/pCGT_pyrH** | 560 ± 25 | 3795 ± 160 | 98% ± 2% |
| W3110lpp3ΔplsC/pCGT_plsC** | 570 ± 30 | 3810 ± 190 | 98% ± 2% |

| | | | |
|---|---|---|---|
| **^{*}Konstrukt im Sinne des Stands der Technik (Vergleichsbeispiel)** ****erfindungsgemäßes Konstrukt** | | | |

### Beispiel 12: Sekretorische, fermentative Produktion des Fab-Antikörperfragments Anti-Lysozym-Fab im 10 1-Maßstab

Mit Hilfe einer *lpp*-Mutante von *E. coli* können auch funktionelle Fab-Antikörperfragmente extrazellulär hergestellt werden (US2008076158A1). Dabei muss die Zelle die entsprechenden Fragmente der leichten Kette, welche die Domänen V_{L} und C_{L} umfasst, und der schweren Kette, welche die Domänen V_{H} und CH1 umfasst, gleichzeitig synthetisieren und dann in das Periplasma und schließlich in das Fermentationsmedium sekretieren. Außerhalb des Cytoplasmas erfolgt dann die Assemblierung der beiden Ketten zum funktionellen Fab-Fragment.

Das vorliegende Beispiel beschreibt die Produktion eines Fab-Fragments des gut charakterisierten Anti-Lysozym-Antikörpers D1.3.

Die Plasmide pFab-anti-Lysozym_tetR, pFab-anti-Lysozym_pyrH und pFab-anti-Lysozym_plsC enthalten neben den Markergenen tetR, *pyrH* bzw. *plsC* u.a. auch die Strukturgene für die HC und die LC des Fab-Fragments in Form eines Operons. Dabei ist die HC *in frame* an das 3'-Ende der ompA-Signalsequenz (ompA^{SS}) und die LC *in frame* an das 3'-Ende einer CGTase-Signalsequenz (cgt^{SS}) anfusioniert. Die Expression des ompA^{SS}-HC-cgt^{SS}-LC-Operons steht unter der Kontrolle des tac-Promotors.

Die Produktion des Anti-Lysozym-Fab-Fragments im 10 1-Maßstab erfolgte analog dem in Beispiel 11 anhand der CGTase beschriebenen Verfahren mit den Stämmen W3110lpp3/pFab-anti-Lysozym_tetR, W3110lpp3ΔpyrH/pFab-anti-Lysozym_pyrH und W3110lpp3ΔplsC/pFab-anti-Lysozym_plsC. Für die Fermentation von *E. coli* W3110lpp3/pFab-anti-Lysozym_tetR im Sinne des Stands der Technik, d.h. mit Antibiotikum als Selektionsmittel, wurde das Medium mit 15 mg/L Tetracyclin supplementiert.

Nach 72 h Fermentation wurden Proben entnommen und anschließend die Zellen durch Zentrifugation vom Fermentationsmedium abgetrennt.

Die Reinigung des Anti-Lysozym-Fab-Fragments aus den Fermentationsüberstanden erfolgte mittels Affinitätschromatographie wie in Skerra (1994, Gene 141, 79-84) beschrieben.

Die Quantifizierung sowie die Bestimmung der Aktivität des gereinigten Anti-Lysozym-Fab-Fragments erfolgte über einen ELISA-Test mit Lysozym als Antigen (Skerra, 1994, Gene 141, 79-84).

In Tabelle 2 sind die hochgerechneten Ausbeuten an funktionellem Anti-Lysozym-Fab-Fragment im Fermentationsüberstand aufgelistet, basierend auf isolierten Mengen aus jeweils 20 ml Fermentationsüberstand nach 72 h Fermentation.

**Tabelle 3: Anti-Lysozym-Fab-Fragment-Ausbeuten im Fermentationsüberstand nach 72 h Fermentation**

| Stamm | Anti-Lysozym-Fab-Fragment-Ausbeute [mg/l] im Fermentationsüberstand (hochgerechnet) | Plasmid-stabilität |
|---|---|---|
| W3110lpp3/pFab-anti-Lysozym_tetR (kultiviert mit Tetracyclin)* | 1440 ± 110 | 97% ± 3% |
| W3110lpp3/pFab-anti-Lysozym_tetR (kultiviert ohne Tetracyclin) | 625 ± 250 | 55% ± 20% |
| W3110lpp3ΔpyrH/pFab-anti-Lysozym pyrH** | 1580 ± 115 | 98% ± 2% |
| W3110lpp3ΔplsC/pFab-anti-Lysozym_plsC** | 1610 ± 130 | 98% ± 2% |

| | | |
|---|---|---|
| **^{*}Konstrukt im Sinne des Stands der Technik (Vergleichsbeispiel)** ****erfindungsgemäßes Konstrukt** | | |

### Beispiel 13: Bestimmung der Plasmidstabilität

Die Plasmidstabilität wurde mittels Plasmidpräparation mit anschließendem Restriktionsverdau überprüft. Hierfür wurden nach Beendigung der Kultivierung der Produktionsstämme (z.B. nach 72 h Fermentation) verschiedene Verdünnungen der Kulturen auf LB-Agarplatten ausplattiert. Für die anschließende Ermittlung der Plasmidstabilität, d.h. die Identifizierung Plasmid-tragender Zellen (Kolonien) wurden nur LB-Platten mit Einzelkolonien für die Auswertung herangezogen.

Insgesamt wurden 50 einzelne Kolonien in flüssigem LB-Medium für 15 bis 20 Stunden kultiviert und anschließend Plasmid-DNA aus diesen Kulturen isoliert. Anhand charakteristischer Restriktionsmuster für die einzelnen Produktionsplasmide wurde die Richtigkeit der isolierten Plasmide verifiziert.

## Patentansprüche

1. Mikroorganismenstamm zur Produktion von niedermolekularen Substanzen oder Proteinen enthaltend in seinem Genom eine Mutation in einem Gen, welche eine Auxotrophie des Stammes bewirkt, sowie ein Produktionsplasmid kodierend mindestens ein Enzym zur Produktion einer niedermolekularen Substanz oder mindestens ein rekombinantes Protein sowie eine funktionelle Kopie des Gens, dessen chromosomale Inaktivierung die Auxotrophie bewirkt, **dadurch gekennzeichnet, dass** es sich bei der Auxotrophie um eine nicht fütterbare Auxotrophie handelt und es sich um das pyrH-Gen mit der SEQ ID Nr. 1 oder ein *pyrH*-homologes Gen handelt,
wobei unter einer nicht fütterbaren Auxotrophie zu verstehen ist, dass die Auxotrophie das Wachstum der Mikroorganismenzelle vermindert oder den Tod der Mikroorganismenzelle bewirkt und nicht durch Zugabe von stoffwechselspezifischen Vor-, Zwischen- und/oder Endprodukten in das Wachstumsmedium supplementierbar ist und wobei es sich bei den pyrH-homologen Genen um Gene handelt, die für ein Protein mit PyrH-Aktivität kodieren und die eine Sequenzidentität größer 30% zu SEQ ID No. 1 aufweisen.

2. Mikroorganismenstamm gemäß Anspruch 1, **dadurch gekennzeichnet, dass** die Mutation des Gens, welches die nicht fütterbare Auxotrophie des Stammes bewirkt, zur Inaktivierung dieses Gens führt.

3. Mikroorganismenstamm gemäß einem der Ansprüche 1 oder 2, **dadurch gekennzeichnet, dass** die Mutation des Gens welches die nicht fütterbare Auxotrophie des Stammes bewirkt, zur Inaktivierung der Aktivität des durch das Gen kodierten Genprodukts führt.

4. Verfahren zur Herstellung eines Stammes gemäß einem der Ansprüche 1 bis 3, **dadurch gekennzeichnet, dass** in einen Mikroorganismenstamm, der das pyrH-Gen oder ein *pyrH-*homologes Gen besitzt, ein temperatursensitives Plasmid eingebracht wird, welches eine funktionelle Kopie des pyrH-Gens oder dessen homologen Gens, das mutiert oder deletiert werden soll, besitzt anschließend das Genom des Stammes derart mutiert wird, dass der Stamm im pyrH Gen oder pyrH-homologen Gen eine Mutation aufweist, die in dem Stamm zu einer nicht-fütterbaren Auxotrophie führt, und in diesem Stamm anschließend das temperatursensitive Plasmid bei nicht-permissiver Temperatur gegen ein Produktionsplasmid ausgetauscht wird, wobei das Produktionsplasmid mindestens ein Gen kodierend ein Enzym zur Produktion einer niedermolekularen Substanz oder mindestens ein rekombinantes Protein sowie eine funktionelle Kopie des pyrH-Gens enthält,
wobei es sich bei *pyrH*-homologen Genen um Gene handelt, die für ein Protein mit PyrH Aktivität kodieren und die eine Sequenzidentität größer 30% zu SEQ ID No. 1 aufweisen

5. Verfahren gemäß Anspruch 4, **dadurch gekennzeichnet, dass** das temperatursensitive Plasmid einen temperatursensitiven Replikationsursprung aufweist.

6. Verfahren gemäß Anspruch 5, **dadurch gekennzeichnet, dass** die Zellen unmittelbar nach der Transformation mit dem Produktionsplasmid für 30-90 min einem Temperaturschock bei 47-55°C ausgesetzt werden und anschließend die weitere Inkubation bei der nicht permissiven Temperatur von 37-45°C erfolgt.

7. Verfahren zur Herstellung von niedermolekularen Substanzen oder Proteinen mittels eines Mikroorganismenstammes in einem Fermentationsmedium, das **dadurch gekennzeichnet ist, dass** ein Mikroorganismenstamm gemäß einem oder mehreren der Ansprüche 1 bis 3 eingesetzt wird und ein antibiotikafreies Fermentationsmedium verwendet wird.

## Claims

1. Microorganism strain for the production of low-molecular-weight substances or proteins, containing in its genome a mutation in a gene, which mutation brings about an auxotrophy in the strain, and also a production plasmid encoding at least one enzyme for the production of a low-molecular-weight substance or at least one recombinant protein and also a functional copy of the gene, the chromosomal inactivation of which brings about the auxotrophy, **characterized in that** the auxotrophy is a nonfeedable auxotrophy and the gene is the pyrH gene having SEQ ID No.1 or a *pyrH*-homologous gene,
wherein a nonfeedable auxotrophy is to be understood to mean that the auxotrophy reduces the growth of the microorganism cell or brings about the death of the microorganism cell and is not supplementable by addition of metabolism-specific precursors, intermediates and/or end products to the growth medium and wherein the pyrH-homologous genes are genes which code for a protein having pyrH activity and which have a sequence identity greater than 30% in relation to SEQ ID No.1.

2. Microorganism strain according to Claim 1, **characterized in that** the mutation of the gene which brings about the nonfeedable auxotrophy in the strain leads to the inactivation of said gene.

3. Microorganism strain according to either of Claims 1 and 2, **characterized in that** the mutation of the gene which brings about the nonfeedable auxotrophy in the strain leads to the inactivation of the activity of the gene product coded by the gene.

4. Method for producing a strain according to any of Claims 1 to 3, **characterized in that** a temperature-sensitive plasmid is introduced into a microorganism strain which has the pyrH gene or a *pyrH*-homologous gene, which temperature-sensitive plasmid has a functional copy of the pyrH gene or the homologous gene thereof that is to be mutated or deleted, then the genome of the strain is mutated such that the strain has in the pyrH gene or pyrH-homologous gene a mutation which leads to a nonfeedable auxotrophy in the strain, and then the temperature-sensitive plasmid is exchanged in said strain at a nonpermissive temperature for a production plasmid, wherein the production plasmid contains at least one gene encoding an enzyme for the production of a low-molecular-weight substance or at least one recombinant protein and also a functional copy of the pyrH gene,
wherein *pyrH*-homologous genes are genes which code for a protein having pyrH activity and which have a sequence identity greater than 30% in relation to SEQ ID No. 1.

5. Method according to Claim 4, **characterized in that** the temperature-sensitive plasmid has a temperature-sensitive origin of replication.

6. Method according to Claim 5, **characterized in that** the cells are exposed, immediately after the transformation with the production plasmid, to a temperature shock at 47-55°C for 30-90 min and the further incubation is then carried out at the nonpermissive temperature of 37-45°C.

7. Method for producing low-molecular-weight substances or proteins by means of a microorganism strain in a fermentation medium, which method is **characterized in that** a microorganism strain according to one or more of Claims 1 to 3 is used and an antibiotic-free fermentation medium is used.

## Revendications

1. Souche de micro-organismes pour la production de substances à bas poids moléculaire ou de protéines contenant dans son génome une mutation dans un gène qui entraîne une auxotrophie dans la souche, ainsi qu'un plasmide de production codant au moins pour une enzyme pour la production d'une substance à bas poids moléculaire ou au moins une protéine recombinante ainsi qu'une copie fonctionnelle du gène, dont l'inactivation chromosomiale entraîne l'auxotrophie, **caractérisée en ce que** l'auxotrophie est une auxotrophie non nourrissable et que le gène est le gène pyrH avec la SEQ ID N°1 ou un gène homologue à *pyrH,*
dans lequel on doit comprendre par auxotrophie non nourrissable que l'auxotrophie réduit la croissance de la cellule de micro-organisme ou entraîne la mort de la cellule de micro-organisme et ne peut pas être supplémentée par l'ajout de précurseurs, d'intermédiaires et/ou de produits finaux spécifiques au métabolisme dans le milieu de croissance et les gènes homologues à pyrH sont des gènes qui codent pour une protéine avec une activité de pyrH et qui présentent une identité de séquence supérieure à 30 % par rapport à SEQ ID N°1.

2. Souche de micro-organismes selon la revendication 1, **caractérisée en ce que** la mutation du gène qui entraîne l'auxotrophie non nourrissable de la souche conduit à l'inactivation de ce gène.

3. Souche de micro-organismes selon la revendication 1 ou 2, **caractérisée en ce que** la mutation du gène qui entraîne l'auxotrophie non nourrissable de la souche conduit à l'inactivation de l'activité du produit génétique codé par le gène.

4. Procédé pour la préparation d'une souche selon l'une quelconque des revendications 1 à 3, **caractérisé en ce que** dans une souche de micro-organismes qui possède le gène pyrH ou un gène homologue à *pyrH,* est introduit un plasmide sensible à la température, qui possède une copie fonctionnelle du gène pyrH ou de son gène homologue, qui doit être muté ou délété, ensuite le génome de la souche est muté de sorte que la souche présente dans le gène pyrH ou un gène homologue à pyrH une mutation qui conduit à une auxotrophie non nourrissable dans la souche, et ensuite le plasmide sensible à la température est remplacé dans cette souche à une température non permissive par un plasmide de production, le plasmide de production contenant au moins un gène codant pour une enzyme pour la production d'une substance à bas poids moléculaire ou d'au moins une protéine recombinante ainsi qu'une copie fonctionnelle du gène pyrH,
les gènes homologue à *pyrH* étant des gènes qui codent pour une protéine dotée d'une activité de pyrH et qui présentent une identité de séquence supérieure à 30 % par rapport à SEQ ID N°1.

5. Procédé selon la revendication 4, **caractérisé en ce que** le plasmide sensible à la température présente une origine de réplication sensible à la température.

6. Procédé selon la revendication 5, **caractérisé en ce que** les cellules sont soumises à un choc thermique de 47 à 55 °C pendant 30 à 90 min immédiatement après la transformation avec le plasmide de production et ensuite l'incubation supplémentaire est réalisée à la température non permissive de 37 à 45 °C.

7. Procédé pour la préparation de substances à bas poids moléculaire ou de protéines au moyen d'une souche de micro-organismes dans un milieu de fermentation, qui est **caractérisé en ce qu'**une souche de micro-organismes selon l'une ou plusieurs des revendications 1 à 3 est utilisée et un milieu de fermentation exempt d'antibiotique est utilisé.


### SEQUENCE LISTING

<110> Consortium für elektrochemische Industrie - Wacker Chemie AG Bornhövd, Dr. Carsten Thön, Dr. Marcel
<120> Mikroorganismenstamm und Verfahren zur antibiotikafreien, fermentativen Herstellung von niedermolekularen Substanzen und Proteinen
<130> Co11517
<150> Co11517
<151> 2015-08-27
<160> 17
<170> PatentIn version 3.5
<210> 1
   <211> 726
   <212> DNA
   <213> Escherichia coli
<220>
   <221> gene
   <222> (1)..(726)
   <223> pyrH
<400> 1
   atggctacca atgcaaaacc cgtctataaa cgcattctgc ttaagttgag tggcgaagct      60
   ctgcagggca ctgaaggctt cggtattgat gcaagcatac tggatcgtat ggctcaggaa     120
   atcaaagaac tggttgaact gggtattcag gttggtgtgg tgattggtgg gggtaacctg     180
   ttccgtggcg ctggtctggc gaaagcgggt atgaaccgcg ttgtgggcga ccacatgggg     240
   atgctggcga ccgtaatgaa cggcctggca atgcgtgatg cactgcaccg cgcctatgtg     300
   aacgctcgtc tgatgtccgc tattccattg aatggcgtgt gcgacagcta cagctgggca     360
   gaagctatca gcctgttgcg caacaaccgt gtggtgatcc tctccgccgg tacaggtaac     420
   ccgttcttta ccaccgactc agcagcttgc ctgcgtggta tcgaaattga agccgatgtg     480
   gtgctgaaag caaccaaagt tgacggcgtg tttaccgctg atccggcgaa agatccaacc     540
   gcaaccatgt acgagcaact gacttacagc gaagtgctgg aaaaagagct gaaagtcatg     600
   gacctggcgg ccttcacgct ggctcgtgac cataaattac cgattcgtgt tttcaatatg     660
   aacaaaccgg gtgcgctgcg ccgtgtggta atgggtgaaa aagaagggac tttaatcacg     720
   gaataa                                                                726
<210> 2
   <211> 241
   <212> PRT
   <213> Escherichia coli
<220>
   <221> PEPTIDE
   <222> (1)..(241)
<400> 2
   Met Ala Thr Asn Ala Lys Pro Val Tyr Lys Arg Ile Leu Leu Lys Leu
   1               5                   10                  15
   Ser Gly Glu Ala Leu Gln Gly Thr Glu Gly Phe Gly Ile Asp Ala Ser
               20                  25                  30
   Ile Leu Asp Arg Met Ala Gln Glu Ile Lys Glu Leu Val Glu Leu Gly
           35                  40                  45
   Ile Gln Val Gly Val Val Ile Gly Gly Gly Asn Leu Phe Arg Gly Ala
       50                  55                  60
   Gly Leu Ala Lys Ala Gly Met Asn Arg Val Val Gly Asp His Met Gly
   65                  70                  75                  80
   Met Leu Ala Thr Val Met Asn Gly Leu Ala Met Arg Asp Ala Leu His
                   85                  90                  95
   Arg Ala Tyr Val Asn Ala Arg Leu Met Ser Ala Ile Pro Leu Asn Gly
               100                 105                 110
   Val Cys Asp Ser Tyr Ser Trp Ala Glu Ala Ile Ser Leu Leu Arg Asn
           115                 120                 125
   Asn Arg Val Val Ile Leu Ser Ala Gly Thr Gly Asn Pro Phe Phe Thr
       130                 135                 140
   Thr Asp Ser Ala Ala Cys Leu Arg Gly Ile Glu Ile Glu Ala Asp Val
   145                 150                 155                 160
   Val Leu Lys Ala Thr Lys Val Asp Gly Val Phe Thr Ala Asp Pro Ala
                   165                 170                 175
   Lys Asp Pro Thr Ala Thr Met Tyr Glu Gln Leu Thr Tyr Ser Glu Val
               180                 185                 190
   Leu Glu Lys Glu Leu Lys Val Met Asp Leu Ala Ala Phe Thr Leu Ala
           195                 200                 205
   Arg Asp His Lys Leu Pro Ile Arg Val Phe Asn Met Asn Lys Pro Gly
       210                 215                 220
   Ala Leu Arg Arg Val Val Met Gly Glu Lys Glu Gly Thr Leu Ile Thr
   225                 230                 235                 240
   Glu
<210> 3
   <211> 738
   <212> DNA
   <213> Escherichia coli
<220>
   <221> gene
   <222> (1)..(738)
   <223> plsC
<400> 3
   atgctatata tctttcgtct tattattacc gtgatttaca gcatcttagt ctgtgtattc     60
   ggctccattt actgcctttt cagcccgcgt aacccgaaac atgtggccac ctttgggcat    120
   atgtttggcc gtcttgcgcc gctgtttggc ctgaaagttg agtgccgtaa acctacagac    180
   gctgaaagct acggcaatgc tatctatatc gctaaccacc agaacaacta tgacatggtg    240
   acagcatcga acatcgtgca accgccgacg gtgacggtag gtaaaaagag cttgctgtgg    300
   atccccttct tcgggcagtt gtactggtta accggcaact tattgatcga cagaaacaat    360
   cgcactaaag ctcacggcac cattgcggaa gtagtgaatc acttcaaaaa acgccgtatt    420
   tccatctgga tgttcccgga aggaacccgc agccgtggtc gcggcctgct accgttcaag    480
   actggagcat ttcacgcggc aattgcggcg ggcgtcccga ttattcccgt gtgcgtctct    540
   acaacttcga ataagattaa tcttaatcga ctgcacaacg gtctggtgat tgtcgaaatg    600
   ctgccgccaa ttgacgtcag tcagtatggc aaagatcagg ttcgtgagct ggctgcccat    660
   tgtcgttcga taatggaaca aaaaatcgcc gagctcgata aagaagtcgc agaacgcgaa    720
   gccgccggaa aagtttaa                                                   738
<210> 4
   <211> 245
   <212> PRT
   <213> Escherichia coli
<220>
   <221> PEPTIDE
   <222> (1)..(245)
<220>
   <221> PEPTIDE
   <222> (1)..(245)
   <223> PlsC
<400> 4
   Met Leu Tyr Ile Phe Arg Leu Ile Ile Thr Val Ile Tyr Ser Ile Leu
   1               5                   10                  15
   Val Cys Val Phe Gly Ser Ile Tyr Cys Leu Phe Ser Pro Arg Asn Pro
               20                  25                  30
   Lys His Val Ala Thr Phe Gly His Met Phe Gly Arg Leu Ala Pro Leu
           35                  40                  45
   Phe Gly Leu Lys Val Glu Cys Arg Lys Pro Thr Asp Ala Glu Ser Tyr
       50                  55                  60
   Gly Asn Ala Ile Tyr Ile Ala Asn His Gln Asn Asn Tyr Asp Met Val
   65                  70                  75                  80
   Thr Ala Ser Asn Ile Val Gln Pro Pro Thr Val Thr Val Gly Lys Lys
                   85                  90                  95
   Ser Leu Leu Trp Ile Pro Phe Phe Gly Gln Leu Tyr Trp Leu Thr Gly
               100                 105                 110
   Asn Leu Leu Ile Asp Arg Asn Asn Arg Thr Lys Ala His Gly Thr Ile
           115                 120                 125
   Ala Glu Val Val Asn His Phe Lys Lys Arg Arg Ile Ser Ile Trp Met
       130                 135                 140
   Phe Pro Glu Gly Thr Arg Ser Arg Gly Arg Gly Leu Leu Pro Phe Lys
   145                 150                 155                 160
   Thr Gly Ala Phe His Ala Ala Ile Ala Ala Gly Val Pro Ile Ile Pro
                   165                 170                 175
   Val Cys Val Ser Thr Thr Ser Asn Lys Ile Asn Leu Asn Arg Leu His
               180                 185                 190
   Asn Gly Leu Val Ile Val Glu Met Leu Pro Pro Ile Asp Val Ser Gln
           195                 200                 205
   Tyr Gly Lys Asp Gln Val Arg Glu Leu Ala Ala His Cys Arg Ser Ile
       210                 215                 220
   Met Glu Gln Lys Ile Ala Glu Leu Asp Lys Glu Val Ala Glu Arg Glu
   225                 230                 235                 240
   Ala Ala Gly Lys Val
                   245
<210> 5
   <211> 40
   <212> DNA
   <213> synthetisches Oligonukleotid
<220>
   <221> misc_feature
   <223> Oligonukleotid: pyrH-NcoI-fw
<400> 5
   ccccccatgg ccatcttgta aattcagcta acccttgtgg 40
<210> 6
   <211> 40
   <212> DNA
   <213> synthetisches Oligonukleotid
<220>
   <221> misc_feature
   <223> Oligonukleotid: pyrH-NcoI-rev
<400> 6
   ggggccatgg atcctcacgt acttttgtac gctccggttg 40
<210> 7
   <211> 37
   <212> DNA
   <213> synthetisches Oligonukleotid
<220>
   <221> misc_feature
   <223> Oligonukleotid: plsC-NcoI-fw
<400> 7
   ccccccatgg atttgctcca tgtaaaactg gctaaag 37
<210> 8
   <211> 40
   <212> DNA
   <213> synthetisches Oligonukleotid
<220>
   <221> misc_feature
   <223> Oligonukleotid: plsC-NcoI-rev
<400> 8
   ggggccatgg tgaaaccgtt gtttattcat gcgttgcgat 40
<210> 9
   <211> 71
   <212> DNA
   <213> synthetisches Oligonukleotid
<220>
   <221> misc_feature
   <223> synthetisches Oligonukleotid: pyrH-fw
<220>
   <221> misc_feature
   <223> Oligonukleotid: pyrH-fw
<400> 9
   cagtcttaat tatcaaaaag gagccgcctg agggcggctt ctttttgtgc cgtgtaggct      60
   ggagctgctt c                                                           71
<210> 10
   <211> 70
   <212> DNA
   <213> synthetisches Oligonukleotid
<220>
   <221> misc_feature
   <223> Oligonukleotid: pyrH-rev
<400> 10
   gtaatcgtct ggattattag gctattttat ttgccatttt ggccccgggc atgggaatta      60
   gccatggtcc                                                             70
<210> 11
   <211> 30
   <212> DNA
   <213> synthetisches Oligonukleotid
<220>
   <221> misc_feature
   <223> Oligonukleotid: pyrH-check-for
<400> 11
   gcataacgct gaagtgactg gcttcatccg 30
<210> 12
   <211> 30
   <212> DNA
   <213> synthetisches Oligonukleotid
<220>
   <221> misc_feature
   <223> Oligonukleotid: pyrH-check-rev
<400> 12
   cagaccagac agtcacacac agtggaagtg 30
<210> 13
   <211> 71
   <212> DNA
   <213> synthetisches Oligonukleotid
<220>
   <221> misc_feature
   <223> Oligonukleotid: plsC-fw
<400> 13
   ctgacgtcaa ttggcggcag catttcgaca atcaccagac cgttgtgcag cgtgtaggct 60
   ggagctgctt c 71
<210> 14
   <211> 71
   <212> DNA
   <213> synthetisches Oligonukleotid
<220>
   <221> misc_feature
   <223> Oligonukleotid: plsC-rev
<400> 14
   ctgacgtcaa ttggcggcag catttcgaca atcaccagac cgttgtgcag cgtgtaggct 60
   ggagctgctt c 71
<210> 15
   <211> 30
   <212> DNA
   <213> synthetisches Oligonukleotid
<220>
   <221> misc_feature
   <223> Oligonukleotid: plsC-check-for
<400> 15
   cgagattcaa gtagcggcgg aacgggtagc 30
<210> 16
   <211> 30
   <212> DNA
   <213> synthetisches Oligonukleotid
<220>
   <221> misc_feature
   <223> Oligonukleotid: plsC-check-rev
<400> 16
   gttaagtcgt catccggcag cgtattcaac 30
<210> 17
   <211> 4487
   <212> DNA
   <213> Plasmid
<220>
   <221> misc_feature
   <222> (1)..(4308)
   <223> Plasmid pMT1
<400> 17
   tcgtgtcgct caaggcgcac tcccgttctg gataatgttt tttgcgccga catcataacg      60
   gttctggcaa atattctgaa atgagctgtt gacaattaat catcggctcg tataatgtgt     120
   ggaattgtga gcggataaca atttcacaca ggaaacagaa ttcccgggga tccgtcgacc     180
   tgcagccaag cttggctgtt ttggcggatg agagaagatt ttcagcctga tacagattaa     240
   atcagaacgc agaagcggtc tgataaaaca gaatttgcct ggcggcagta gcgcggtggt     300
   cccacctgac cccatgccga actcagaagt gaaacgccgt agcgccgatg gtagtgtggg     360
   gtctccccat gcgagagtag ggaactgcca ggcatcaaat aaaacgaaag gctcagtcga     420
   aagactgggc ctttcgtttt atctgttgtt tgtcggtgaa cgctctcctg agtaggacaa     480
   atccgccggg agcggatttg aacgttgcga agcaacggcc cggagggtgg cgggcaggac     540
   gcccgccata aactgccagg catcaaatta agcagaaggc catcctgacg gatggccttt     600
   ttgcgtttct acaaactctt ttgtttattt ttctaaatac attcaaatat gtatccgctc     660
   atgagacaat aaccctgata aatgcttcaa taatattgaa aaaggaagag tatgagtatt     720
   cggtcgccgc atacactatt ctcagaatga cttggttgag tccatggcgg ccgccgatct     780
   aggtgaagat cctttttgat aatctcatga ccaaaatccc ttaacgtgag ttttcgttcc     840
   actgagcgtc agaccccgta gaaaagatca aaggatcttc ttgagatcct ttttttctgc     900
   gcgtaatctg ctgcttgcaa acaaaaaaac caccgctacc agcggtggtt tgtttgccgg     960
   atcaagagct accaactctt tttccgaagg taactggctt cagcagagcg cagataccaa    1020
   atactgtcct tctagtgtag ccgtagttag gccaccactt caagaactct gtagcaccgc 1080
   ctacatacct cgctctgcta atcctgttac cagtggctgc tgccagtggc gataagtcgt 1140
   gtcttaccgg gttggactca agacgatagt taccggataa ggcgcagcgg tcgggctgaa 1200
   cggggggttc gtgcacacag cccagcttgg agcgaacgac ctacaccgaa ctgagatacc 1260
   tacagcgtga gcattgagaa agcgccacgc ttcccgaagg gagaaaggcg gacaggtatc 1320
   cggtaagcgg cagggtcgga acaggagagc gcacgaggga gcttccaggg ggaaacgcct 1380
   ggtatcttta tagtcctgtc gggtttcgcc acctctgact tgagcgtcga tttttgtgat 1440
   gctcgtcagg ggggcggagc ctatggaaaa acgattctca tgtttgacag cttatcatcg 1500
   ataagctcca ggacccaacg ctgcccgaga tgcgccgcgt gcggctgctg gagatggcgg 1560
   acgcgatgga tatgttctgc caagggttgg tttgcgcatt cacagttctc cgcaagaatt 1620
   gattggctcc aattcttgga gtggtgaatc cgttagcgag gtgccgccgg cttccattca 1680
   ggtcgaggtg gcccggctcc atgcaccgcg acgcaacgcg gggaggcaga caaggtatag 1740
   ggcggcgcct acaatccatg ccaacccgtt ccatgtgctc gccgaggcgg cataaatcgc 1800
   cgtgacgatc agcggtccaa tgatcgaagt taggctggta agagccgcga gcgatccttg 1860
   aagctgtccc tgatggtcgt catctacctg cctggacagc atggcctgca acgcgggcat 1920
   cccgatgccg ccggaagcga gaagaatcat aatggggaag gccatccagc ctcgcgtcgc 1980
   gaacgccagc aagacgtagc ccagcgcgtc ggccgccatg ccggcgataa tggcctgctt 2040
   ctcgccgaaa cgtttggtgg cgggaccagt gacgaaggct tgagcgaggg cgtgcaagat 2100
   tccgaatacc gcaagcgaca ggccgatcat cgtcgcgctc cagcgaaagc ggtcctcgcc 2160
   gaaaatgacc cagagcgctg ccggcacctg tcctacgagt tgcatgataa agaagacagt 2220
   cataagtgcg gcgacgatag tcatgccccg cgcccaccgg aaggagctga ctgggttgaa 2280
   ggctctcaag ggcatcggtc gacgctctcc cttatgcgac tcctgcatta ggaagcagcc 2340
   cagtagtagg ttgaggccgt tgagcaccgc cgccgcaagg aatggtgcat gcaaggagat 2400
   ggcgcccaac agtcccccgg ccacggggcc tgccaccata cccacgccga aacaagcgct 2460
   catgagcccg aagtggcgag cccgatcttc cccatcggtg atgtcggcga tataggcgcc 2520
   agcaaccgca cctgtggcgc cggtgatgcc ggccacgatg cgtccggcgt agaggatcca 2580
   caggacgggt gtggtcgcca tgatcgcgta gtcgatagtg gctccaagta gcgaagcgag 2640
   caggactggg cggcggccaa agcggtcgga cagtgctccg agaacgggtg cgcatagaaa 2700
   ttgcatcaac gcatatagcg ctagcagcac gccatagtga ctggcgatgc tgtcggaatg 2760
   gacgatatcc cgcaagaggc ccggcagtac cggcataacc aagcctatgc ctacagcatc 2820
   cagggtgacg gtgccgagga tgacgatgag cgcattgtta gatttcatac acggtgcctg    2880
   actgcgttag caatttaact gtgataaact accgcattaa agcttatcga tgataagctg    2940
   tcaaacatgg caggcctccc ccagaagtcg cttgcggtat tcggaatctt gcacgccctc    3000
   gctcaagcct tcgtcactgg tcccgccacc aaacgtttcg gcgagaagca ggccattatc    3060
   gccggcatgg cggccgacgc gctgggctac gtcttgctgg cgttcgcgaa cgccagcaag    3120
   acgtagccca gcgcgtcggc cagcttgcaa ttcgcgctaa cttacattaa ttgcgttgcg    3180
   ctcactgccc gctttccagt cgggaaacct gtcgtgccag ctgcattaat gaatcggcca    3240
   acgcgcgggg agaggcggtt tgcgtattgg gcgccagggt ggtttttctt ttcaccagtg    3300
   agacgggcaa cagctgattg cccttcaccg cctggccctg agagagttgc agcaagcggt    3360
   ccacgctggt ttgccccagc aggcgaaaat cctgtttgat ggtggttgac ggcgggatat    3420
   aacatgagct gtcttcggta tcgtcgtatc ccactaccga gatatccgca ccaacgcgca    3480
   gcccggactc ggtaatggcg cgcattgcgc ccagcgccat ctgatcgttg gcaaccagca    3540
   tcgcagtggg aacgatgccc tcattcagca tttgcatggt ttgttgaaaa ccggacatgg    3600
   cactccagtc gccttcccgt tccgctatcg gctgaatttg attgcgagtg agatatttat    3660
   gccagccagc cagacgcaga cgcgccgaga cagaacttaa tgggcccgct aacagcgcga    3720
   tttgctggtg acccaatgcg accagatgct ccacgcccag tcgcgtaccg tcttcatggg    3780
   agaaaataat actgttgatg ggtgtctggt cagagacatc aagaaataac gccggaacat    3840
   tagtgcaggc agcttccaca gcaatggcat cctggtcatc cagcggatag ttaatgatca    3900
   gcccactgac gcgttgcgcg agaagattgt gcaccgccgc tttacaggct tcgacgccgc    3960
   ttcgttctac catcgacacc accacgctgg cacccagttg atcggcgcga gatttaatcg    4020
   ccgcgacaat ttgcgacggc gcgtgcaggg ccagactgga ggtggcaacg ccaatcagca    4080
   acgactgttt gcccgccagt tgttgtgcca cgcggttggg aatgtaattc agctccgcca    4140
   tcgccgcttc cactttttcc cgcgttttcg cagaaacgtg gctggcctgg ttcaccacgc    4200
   gggaaacggt ctgataagag acaccggcat actctgcgac atcgtataac gttactggtt    4260
   tcacattcac caccctgaat tgactctctt ccgggcgcta tcatgccata ccgcgaaagg    4320
   ttttgcacca ttcgatggtg tcaacgtaaa tgcatgccgc ttcgccttcg cgcgcgaatt    4380
   gcaagctgat ccgggcttat cgactgcacg gtgcaccaat gcttctggcg tcaggcagcc    4440
   atcggaagct gtggtatggc tgtgcaggtc gtaaatcact gcataat                   4487